# EUROPÄISCHE PATENTSCHRIFT

(11) **EP 0 605 797 B1**
(45) Veröffentlichungstag und Bekanntmachung des Hinweises auf die Patenterteilung: **17.03.1999**
(21) Anmeldenummer: 93119574.7
(22) Anmeldetag: 09.12.1993
(51) Int. Cl.: C07K 14/75, G01N 33/06

(54) **Synthetische Peptide, Antikörper dagegen und ihre Verwendung**
Synthetic peptides, antibodies directed against them and their use
Peptides synthétiques, des anticorps contrariés et leur utilisation

(30) Priorität: 17.12.1992 DE 4242736
(43) Veröffentlichungstag der Anmeldung: 13.07.1994
(73) Patentinhaber: Dade Behring Marburg GmbH, 35001 Marburg (DE)
(72) Erfinder: Kraus, Michael, D-35041 Marburg (DE); Stüber, Werner, D-35094 Lahntal 3 (DE)

(56) Entgegenhaltungen:
- J.BIOL.CHEM. Bd. 254, Nr. 3 , 1979 , USA Seiten 672 - 678 E.F.PLOW ET AL. 'Localization and Characterization of the Cleavage-associated Neoantigen Locus in the E Domain of Fibrinogen'
- BRITHISH JOURNAL OF HAEMATOLOGY Bd. 57 , 184 Seiten 133 - 144 J.P.CHEN ET AL. 'Radioimmunoessay of fragment E-related neoantigen: validation studies and clinical application'
- THROMBOSIS AND HAEMOSTASIS Bd. 59, Nr. 2 , 1988 , STUTTGART Seiten 310 - 315 P.W.KOPPERT ET AL. 'A Monoclonal Antibody-Based Enzyme Immunoessay for Fibrinogen Degradation Products in Plasma'
- ADV.EXP.MED.BIOL. Bd. 281 , 1990 , NEW YORK Seiten 39 - 48 D:W:CHUNG ET AL. 'Nucleotide Sequences of the Three Genes coding for Human Fibrinogen'
- J.BIOL.CHEM. Bd. 265, Nr. 23 , 1990 , USA Seiten 13669 - 13676 N.E.KIRSCHBAUM ET AL. 'A Unique Proteolytic Fragment of Human Fibrinogen Containing the Aalpha COOH- terminal Domain of the Native Molecule'

## Beschreibung

Die Erfindung bezieht sich auf Peptide, mit deren Hilfe erzeugte Antikörper sowie die Verwendung solcher Peptide und Antikörper zu therapeutischen und diagnostischen Zwecken.

Der Organismus ist durch das Gerinnungssystem vor Blutverlust geschützt. Die Gerinnungskaskade mündet in der Aktivierung der Protease Thrombin, die unter Abspaltung der Fibrinopeptide A und B Fibrinogen zu Fibrin umsetzt. Die einzelnen Fibrinmoleküle lagern sich aneinander an (sogenannter "weicher Clot") und werden im Normalfall durch die Transpeptidase Faktor XIII untereinander quervernetzt (sogenannter "harter Clot"). Dieser Wundverschluß wird durch das im Gegenzug aktivierte fibrinolytische System aufgelöst. Das Schlüsselenzym der Fibrinolyse ist die Protease Plasmin, die Fibrinogen und Fibrin im wesentlichen in die Fragmente D und E spaltet. Fibrinogen ist symmetrisch aus 2 Tripeptiden aufgebaut, die nahe der N-Termini über Disulfidbrücken miteinander verknüpft sind. Bei der Spaltung von Fibrin bzw. Fibrinogen entsteht daher pro Molekül 1 Molekül Fragment E, das die zentrale Verknüpfungsregion des Fibrin(ogen)moleküls umfaßt, sowie 2 Moleküle Fragment D. In einem harten Clot sind die D-Domänen des Fibrins quervernetzt, so daß der Abbau durch Plasmin D-Dimer und Fragment E freisetzt. Das Fragment E selbst unterliegt zwei weiteren Abbaustufen. In seiner ersten und zweiten Form (E1 bzw. E2) ist es an D-Dimer nicht-kovalent gebunden und bildet den DD/E-Komplex. Erst nach dem zweiten enzymatischen Abbau dissoziiert das Fragment E3 vom D-Dimer-Molekül.

Proteine, die entweder innerhalb eines Moleküls mehrere immunchemisch identische Epitope tragen oder Proteine wie das D-Dimer, das bei der Spaltung von Fibrin bzw. Fibrinogen entsteht, die zumindest unter physiologischen Bedingungen als Oligomere aus Proteinmolekülen zusammengesetzt sind, die jeweils mindestens ein immunchemisch identisches Epitop tragen, werden auch als "intramolekulare Oligomere" bezeichnet.

In vielen pathologischen Situationen kann es im Gefäßsystem zu einer unerwünschten Aktivierung des Gerinnungssystems mit nachfolgendem Verschluß kommen. Dies kann zu schweren Herzattacken und Thromboembolien führen. Bei Patienten, die auf Grund dieser hypercoagulatorischen Zustände mit Thrombolytika behandelt werden, muß der Lysiserfolg zur Kontrolle der Therapie verfolgt werden. Dies wird durch die Bestimmung des D-Dimers erreicht. Die Thrombolytika sind jedoch nicht spezifisch, so daß durch die systemische Aktivierung des Plasmins auch Fibrinogen verstärkt abgebaut wird. Dieser Fibrinogenabbau könnte rechtzeitig durch Bestimmung des Fragment E nachgewiesen werden. Auch bei hyperfibrinolytischen Zustände, ausgelöst etwa bei Sepsis über das Komplementsystem, die z. B. zur Entwicklung einer disseminierten intravasalen Gerinnung (DIC) führen können, wird vorwiegend Fibrinogen abgebaut. Ein Fibrinogenverbrauch birgt aber ein erhöhtes Blutungsrisiko, das mit der Bestimmung von Fragment E somit rechtzeitig diagnostisch erkannt und dem dadurch therapeutisch entgegengesteuert werden kann.

Zum Nachweis von Spaltprodukten des Fibrin(ogen)s sind zahlreiche Verfahren bekannt, so z. B. der Hemaglutinations Inhibierungstest (Mersky C. et al., "A rapid, simple, sensitive method for measuring fibrinolytic split products in human serum"; Proc. Soc. Exp. Biol. Med. 131: 871-875 (1969)). Dieses Prinzip wurde von Schifreen et al., "A quantitative automated immunoassay for fibrinogen/fibrin degradation products", Clin. Chem. 31: 1468-1473 (1985) übernommen, wobei die Erythrozyten durch Latexpartikel ersetzt wurden.

Andere Aggregationsassays zur Bestimmung von Fibrin(ogen)spaltprodukten benutzen Latexpartikel, die mit Antikörpern gegen Fibrin(ogen)spaltprodukte beschichtet sind. Die bekannten Antikörper wurden durch Immunisierung mit den nativen Spaltprodukten erzeugt. Zur Anwendung kamen Antikörper mit verschiedenen Spezifitäten.

Assays, die polyklonale Antikörper oder Fibrinogenrezeptoren verwenden, ist gemeinsam, daß eine Kreuzreaktivität mit Fibrinogen besteht. Durch die daher notwendige Probenvorbehandlung enthalten die Proben unterschiedliche Mengen an Fibrinogen und artifiziell erzeugten Spaltprodukten, so daß diese Methoden allenfalls semiquantitative Aussagen gestatten (Gaffney P.J. and Perry M.J., "Unreliabilitiy of current serum degradation products (FDP) assays", Thromb. Haemost. 53: 301-302 (1985); Nieuwenhuizen W., "Plasma assays of fibrinogen/fibrin degradation products and their clinical relevance", in: Fibrinogen 2. Biochemistry, Physiology and Clinical Relevance. G.D.O. Lowe et al, Edt., 173-180 (1987)).

Auch Assays, die monoklonale Antikörper verwenden, vermeiden auf Grund der Spezifität der Antikörper die Problematik der Kreuzreaktivität mit intaktem Fibrinogen bzw. Fibrin. Für die Anwendung in Agglutinationsassays muß aber zur Bildung von Aggregaten das detektierte Epitop zweimal auf dem Antigen für die Antikörper zugänglich sein. Daher erkennen z. B. die oben erwähnten Latexassays, die monoklonale Antikörper gegen D-Monomer verwenden (z. B. Patent App. WO 86/01298) nicht D-Monomer, sondern D-Dimer, das nur nach Quervernetzung aus Fibrin hervorgeht (siehe Gaffney P.J. et al., "Monoclonal antibodies against fibrinogen, fibrin and their fragments.", Thromb. Haemost. 54: 733-734 (1985)). Diese Tests sind somit nicht zur Erfassung von Fibrin(ogen)spaltprodukten geeignet.

Neben diesen homogenen Testverfahren wurde in jüngster Zeit ein ELISA beschrieben, in dem mittels monoklonaler Antikörper zwischen Fibrinogen und Fibrinspaltprodukten differenziert werden kann (Koppert P.W. et al., "A monoclonal antibody-based enzyme immunoassay for fibrin degradation products in plasma", Thromb. Haemostas. 59: 310-315 (1988)).

Aus prinzipiellen Gründen sind allerdings die bekannten ELISA-Verfahren im Vergleich zu homogenen Verfahren arbeitsintensiver, zeitaufwendiger und in der Regel schlechter automatisierbar.

Aus der DE 37 01 812 ist die Verwendung eines Hexapeptids des N-Terminus der α-Kette des Fibrins, das durch Einwirkung von Thrombin entsteht, bekannt.

Hui K.Y. et al. ("Monoclonal antibodies to a synthetic fibrin-like peptide bind to human fibrin but not fibrinogen", Science 222: 1129-1132 (1983)) verwendete ein Heptapeptid aus dem entsprechenden N-Terminus der β-Kette. Die mit diesen Methoden erhaltenen Antikörper erkennen nur Fibrin und keine Fibrin(ogen)spaltprodukte. Diese Assays und auch der Nachweis der Fibrinopeptide A und B, die bei der Umwandlung von Fibrinogen zu Fibrin durch Thrombin freigesetzt werden, dienen der Diagnose hypercoagulatorischer und nicht der hyperfibrinolytischer Zustände.

Zum Nachweis von Fibrin(ogen)spaltprodukten in menschlichem Blut, Synovialflüssigkeit oder Urin sind spezifische Antikörper notwendig, die ausschließlich mit Fragment E reagieren, nicht jedoch das native Fibrinogen oder Fibrin erkennen. Diese Antikörper sollen zudem einfach zu erhalten sein und in allen bekannten immunchemischen Verfahren also sowohl heterogenen, als auch homogenen Testmethoden verwendet werden können.

Der vorliegenden Erfindung lag daher die Aufgabe zugrunde, ein Antigen zur Verfügung zu stellen, das zur Bildung von Antikörpern gegen Fibrinogen- und Fibrinspaltprodukten führt, die leicht zu reinigen und spezifisch sind und somit eine exakte Quantifizierung der fibrinolytischen Aktivität in biologischen Flüssigkeiten, unabhängig vom Gehalt an Fibrinogen oder Fibrin, ermöglichen. Weiterhin sollten diese Antikörper neben heterogenen Assays auch die Anwendung homogener Immunoassaytechniken ermöglichen.

Überraschenderweise wurde gefunden, daß durch Immunisierung von Tieren mit synthetischen Peptiden aus den C-terminalen Regionen des Fragments E Antikörper erhalten werden können, die nicht mit Fibrinogen oder Fibrin, aber spezifisch mit allen 3 Fragmenten E reagieren und zudem die Verwendung in Agglutinationsassays erlauben.

Gegenstand der Erfindung sind daher synthetische Peptide, die Aminosäuresequenzen aufweisen, die zumindest teilweise den durch Plasminspaltung von Fibrinogen entstehenden carboxyterminalen Enden des Fragments E entsprechen und antigen sind, bevorzugterweise enthalten sie mindestens eine der folgenden Aminosäuresequenzen: 
a) Leu-Phe-Glu-Tyr-Gln-Lys-OH,
b) Tyr-Met-Tyr-Leu-Leu-Lys-OH,
c) Val-Lys-Glu-Leu-Ile-Lys-OH und
d) His-Gln-Val-Glu-Asn-Lys-OH,
 besonders bevorzugterweise mindestens eine der folgenden Aminosäuresequenzen: 
a) Asn-Lys-Leu-Lys-Asn-Ser-Leu-Phe-Glu-Tyr-Gln-Lys-OH,
b) Ser-Ser-Ser-Ser-Phe-Gln-Tyr-Met-Tyr-Leu-Leu-Lys-OH,
c) Glu-Asn-Lys-Thr-Ser-Gln-Val-Lys-Gln-Leu-Ile-Lys-OH und
d) Ser-Leu-Glu-Asp-Ile-leu-His-Gln-Val-Glu-Asn-Lys-OH.
 ganz besonders bevorzugterweise mindestens eine der folgenden Aminosäuresequenzen:

a) His-Gln-Ser-Ala-Cys-Lys-Asp-Ser-Asp-Trp-Pro-Phe-Cys-
Ser-Asp-Glu-Asp-Trp-Asn-Tyr-Lys-Cys-Pro-Ser-Gly-Cys-
Arg-Met-Lys-Gly-Leu-Ile-Asp-Glu-Val-Asn-Gln-Asp-Phe-
Thr-Asn-Arg-Ile-Asn-Lys-Leu-Lys-Asn-Ser-Leu-Phe-Glu-
Tyr-Gln-Lys-OH (Peptid 1)

b) Lys-Val-Glu-Arg-Lys-Ala-Pro-Asp-Ala-Gly-Gly-Cys-Leu-
His-Ala-Asp-Pro-Asp-Leu-Gly-Val-Leu-Cys-Pro-Thr-Gly-
Cys-Gln-Leu-Gln-Glu-Ala-Leu-Leu-Gln-Gln-Glu-Arg-Pro-
Ile-Arg-Asn-Ser-Val-Asp-Glu-Leu-Asn-Asn-Asn-Val-Glu-
Ala-Val-Ser-Gln-Thr-Ser-Ser-Ser-Ser-Phe-Gln-Tyr-Met-
Tyr-Leu-Leu-Lys-OH (Peptid 2)

c) Tyr-Val-Ala-Thr-Arg-Asp-Asn-Cys-Cys-Ile-Leu-Asp-Glu-
Arg-Phe-Gly-Ser-Tyr-Cys-Pro-Thr-Thr-Cys-Gly-Ile-Ala-
Asp-Phe-Leu-Ser-Thr-Tyr-Gln-Thr-Lys-Val-Asp-Lys-Asp-
Leu-Gln-Ser-Leu-Glu-Asp-Ile-Leu-His-Gln-Val-Glu-Asn-
Lys-Thr-Ser-Glu-Val-Lys-Gln-Leu-Ile-Lys-OH (Peptid 3)
und

d) Tyr-Val-Ala-Thr-Arg-Asp-Asn-Cys-Cys-Ile-Leu-Asp-Glu-
Arg-Phe-Gly-Ser-Tyr-Cys-Pro-Thr-Thr-Cys-Gly-Ile-Ala-
Asp-Phe-Leu-Ser-Thr-Tyr-Gln-Thr-Lys-Val-Asp-Lys-Asp-
Leu-Gln-Ser-Leu-Glu-Asp-Ile-Leu-His-Gln-Val-Glu-Asn-
Lys (Peptid 4).

Bevorzugt sind auch Peptide, die aus den Peptid 1 - 3 entsprechenden Aminosäuresequenzen bestehen.

Die erfinderischen Peptide können auch direkt oder über einen Spacer an ein Trägermolekül gebunden sein.

Sie werden gentechnisch oder durch chemische Synthese hergestellt.

Gegenstand der Erfindung sind ferner Antikörper, die mit den erfindungsgemäßen Peptiden immunchemisch reagieren.

Solche Antikörper werden durch Immunisierung eines Tieres mit erfindungsgemäßen Peptid erhalten.

Die erfindungsgemäßen Antikörper werden bevorzugterweise, insbesondere wenn sie polyklonal sind, über Immunadsorption an erfindungsgemäßen Peptiden isoliert und gereinigt.

Die erfindungsgemäßen Antikörper können auch bevorzugterweise nach dem Fachmann bekannten Verfahren hergestellte monoklonale Antikörper sein.

Gegenstand der Erfindung sind auch diagnostische Verfahren zur immunchemischen Bestimmung von intramolekularen Oligomeren aus Fibrinogen- und Fibrinspaltprodukten, unter Verwendung erfindungsgemäßer Peptide und/oder erfindungsgemäße Antikörper, insbesondere zur Bestimmung von Fibrin(ogen) Fragmenten E und D-Dimer.

Bevorzugt ist dabei ein heterogener Immunoassay, besonders bevorzugterweise ein Enzymimmunoassay.

Bevorzugterweise wird auch ein homogener Immunoassay, besonders bevorzugterweise ein partikelverstärkter, nephelometrischer oder turbidimetrischer Test verwendet.

Bei dem Verfahren ist vorteilhafterweise ein Teil der Antikörper an eine Festphase gebunden und der andere Teil trägt eine nachweisbare Funktion, wobei ein Verfahren bevorzugt ist, das als Festphase Mikrotitrationsplatten verwendet und die nachweisbare Funktion ein fluorogener oder lumineszenter Farbstoff oder ein Enzym ist.

Bei dem homogenen Immunoassay wird vorteilhafterweise als Festphase ein partikulärer, wasserunlöslicher Träger verwendet und die Agglutinationsreaktion wird nephelometrisch oder turbidimetrisch gemessen.

Bei heterogenen und homogenen Verfahren wird vorteilhafterweise nur eine monospezifische Spezies verwendet, wobei allerdings auch ein Verfahren vorteilhaft ist, bei dem der oder die Fängerantikörper erfindungsgemäße Antikörper sind, während der oder die Detektionsantikörper davon verschieden sein können.

Gegenstand der Erfindung ist außerdem die Verwendung von erfindungsgemäßen Peptiden zu therapeutischen Zwecken, insbesondere zur Therapie von Störungen des fibrinolytischen Systems.

Gegenstand der Erfindung ist ferner die Verwendung von erfindungsgemäßen Antikörpern zu therapeutischen Zwecken, inbesondere zur Therapie von Störungen des fibrinolytischen Systems.

Gegenstand der Erfindung ist darüber hinaus ein immunchemisches Verfahren zur Bestimmung von intramolekularen Oligomeren, in dem nur eine monospezifische Antikörperspezies verwendet wird.

Das Verfahren kann ein heterogener Immunoassay, bevorzugterweise ein Enzymimmunoassay, oder ein homogener Immunoassay, bevorzugterweise ein partikelverstärkter, nephelometrischer oder turbidimetrischer Test sein.

Bei dem heterogenen Immunoassay ist vorteilhafterweise ein Teil der Antikorper an eine Festphase gebunden, und der andere Teil trägt eine nachweisbare Funktion.

Bevorzugt wird dabei als Festphase eine Mikrotitrationsplatte verwendet und die nachweisbare Funktion ist ein fluorogener oder lumineszenter Farbstoff oder ein Enzym.

Ohne damit einen bestimmten Wirkungsmechanismus festlegen zu wollen, scheint die Annahme berechtigt, daß für die erfindungsgemäßen Peptide ein Lysin am carboxyterminalen Ende von Bedeutung ist.

Die erfindungsgemäßen Peptide können nach dem Fachmann an sich bekannten Verfahren hergestellt werden, z. B. (Beispiel 1) können dabei geschützte Aminosäurederivate oder Peptidsegmente in Lösung oder an einer Festphase aneinander gekoppelt werden und durch Abspalten der Schutzgruppen, sowie im Falle einer Festphase durch Abspaltung vom Trägerharz, erfindungsgemäße Peptide erhalten werden. Als temporäre Schutzgruppe werden dabei bevorzugterweise die Fmoc-Gruppe, die permanenten Schutzgruppen für die Seitenfunktionen auf t-Butyl/Boc-Basis, für Arg die Pmc- oder Mtr-Gruppe und für Cys die tert.- Butylmercaptogruppen oder Tritylgruppen benutzt. Die Immobilisierung der C-terminalen Aminosäure erfolgt über p-Alkoxybenzylestergruppen, die an einen üblicherweise für die Peptidsynthese geeigneten polymeren Träger, vorzugsweise quervernetztem Polystyrol, gebunden sind. Der Peptidaufbau erfolgt unter repetitiver Fmoc-Abspaltung, vorzugsweise mit 20 % Piperidin in DMF (Dimethylformamid) (v/v) und Kopplung der folgenden, geschützten Aminosäure, vorzugsweise mit einem Carbodiimid in Gegenwart von HOBT. Das Aminosäurederivat wird hierzu in einem Überschuß, vorzugsweise 3fach, während 1 - 1.5 h in DMF gekoppelt. Nach jedem Arbeitsschritt, Fmoc-Abspaltung bzw. Kondensationsschritt, wird das Harz mit kleinen (15 ml/g) Portionen DMF bzw. Isopropanol je 3mal gewaschen. Die Abspaltung der erfindungsgemäßen Peptide erfolgt acidolytisch unter gleichzeitiger Freisetzung der Seitenkettenfunktionen. Gegebenenfalls freizulegende Sulfhydrylgruppen werden mit Tri-n-Butylphosphin in einem Alkohol, beispielsweise Trifluorethanol oder mit DTT in Wasser "entschützt". Im Falle der Cys (Trt)-Entschützung ist ein separater Arbeitsschritt bei Benutzung von Ethandithiol als Scavenger unnötig. Die Reinigung der Peptide kann z. B. erfolgen über Ionenaustauschchromatographie, reversed-phase Chromatographie und Gelpermeationschromatographie. Die korrekte Zusammensetzung der Peptide sowie die Peptidgehalte werden durch Aminosäureanalyse bestimmt.

Antikörper, die gegen ein Peptid oder Polypeptid gerichtet sind, das dem Bereich der -Kette des Fibrinogens von Aminosäure 1 bis 62 entsprechen, reagieren spezifisch mit den verschiedenen Formen des Fragments E (siehe auch Beispiel 7). Zur Immunisierung eignen sich sowohl die kompletten Polypeptide aus den genannten Bereichen, als auch Teilsequenzen dieser Peptide. Eine besonders bevorzugte Ausführungsform sieht die Verwendung von Octadecapeptiden vor, beispielsweise mit den Sequenzen Val-Asp-Lys-Asp-Leu-Gln-Ser-Leu-Glu-Asp-Ile-Leu-His-Gln-Val-Glu-Asn-Lys aus dem C-Terminus der -Kette des Fragments E.

Für die genannten Fälle ist es wichtig, daß die carboxy-terminale Sequenz des Moleküls exponiert ist und zur Immunisierung führt.

In Anbetracht der für die Peptide vorgesehenen Verwendung ist es sinnvoll, Aminosäuren mit reaktiven Seitengruppen so in die Peptide einzuführen, daß sie die Struktur des Haptens nicht beeinflussen. Zweckmäßigerweise wird aus diesem Grund N-terminal gegebenenfalls Cystein angefügt, dessen freie SH-Gruppe zur Kopplung via Thioether an viele Träger geeignet ist. Bevorzugt wird z. B. das durch das oben genannte Peptid repräsentierte Antigen in Form des Nonadecapeptids Cys-Val-Asp-Lys-Asp-Leu-Gln-Ser-Leu-Glu-Asp-Ile-Leu-His-Gln-Val-Glu-Asn-Lys zur Verfügung gestellt.

Die Herstellung des zur Immunisierung eingesetzten Peptides kann sowohl auf dem Fachmann an sich bekannte Art durch chemische Synthese geschehen, als auch durch Reinigung eines gentechnologisch bereitgestellten Polypeptides oder durch Reinigung eines Peptids, das biochemisch aus Fragment E durch Einwirkung von Proteasen und/ oder chemisch durch Einwirkung von Peptidketten spaltenden Reagenzien, wie z. B. Bromcyan, erhalten wurde.

Peptide, die zur Immunisierung eingesetzt werden sollen oder solche, die Verwendung als Immunoadsorbens finden sollen, werden sinnvollerweise an ein Trägermolekül gekoppelt. Kopplungsverfahren sind dem Fachmann an sich bekannt und in der Literatur beschrieben (Nakane, P.K. et al., "Peroxidase-Labeled Antibody - A New Method of Conjugation", J. Histochem. Cytochem, 22: 1084-1091 (1974), Freifelder, D.M., "Physical Biochemistry." W.H. Freeman and Co., 1976). Trägermoleküle im Sinne dieser Erfindung können sein: natürliche oder synthetische Makromoleküle wie sie der Fachmann zur Erzeugung eines immunreaktiven Konjugats verwendet, z. B. Albumin, Ovalbumin, Keyhole Limpet Hemocyanine oder Polysaccharide. In einer bevorzugten Ausführungsform wird das Peptid oder Polypeptid an das Keyhole Limpet Hemocyanin gebunden.

Bei Verwendung der erfindungsgemäßen synthetischen Peptide als Immunadsorbens empfiehlt sich die Kopplung an Materialien, die sich zum Bereitstellen fester Matrizes eignen. Trägermoleküle in diesem Sinne sind unlösliche Polymere, wie sie der Fachmann zur Immobilisierung von Proteinen und Peptiden verwendet, wie z. B. Polystyrol, Nylon, Agarose oder magnetisierbare Partikel. Die feste Phase kann dabei in beliebiger Form z. B. als Röhrchen, Vlies, Kugel, Faser oder Mikropartikel vorliegen.

Eine bevorzugte Ausführungsform sieht die Kopplung von Peptiden, z. B. des oben erwähnten Nonadecapeptids, an bromcyanaktivierte Sepharose vor.

Die Immunisierung geeigneter Tiere mit trägergebundenen Peptiden führt reproduzierbar zur Bildung von Antikörpern. Eine bevorzugte Tierspezies für Immunisierung und Antikörpergewinnung ist hierbei das Kaninchen; darüber hinaus können auch Mäuse zur Immunisierung verwendet werden.

Aus einem solchen, erfindungsgemäß mit synthetischen Peptiden im Tier erzeugten, Antiserum kann die für spezifische Tests relevante Immunglobulinfraktion durch übliche immunadsorptive Methoden angereichert werden. Bevorzugt ist es in diesem Fall jedoch, als Material für eine solche zur Immunadsorption eingesetzte Matrix ebenfalls ein an einen Träger gekoppeltes Peptid zu verwenden, das die gleiche antigene Determinante wie das zur Immunisierung eingesetzte Peptid aufweist. Das zur immunadsorptiven Reinigung verwendete Peptid kann auch eine verkürzte Aminosäuresequenz aufweisen; Voraussetzung zur Verwendung in der immunadsorptiven Reinigung des gewünschten Antikörpers ist lediglich, daß die von diesem kürzeren Polypeptid gebildete, antigene Determinante vom gewünschten Antikörper erkannt und effektiv gebunden wird.

Das für die immunadsorptive Gewinnung der Antikörper verwendete Peptid kann z. B. ein Nonadekapeptid sein, bevorzugt ist das Peptid Cys-Val-Asp-Lys-Asp-Leu-Gln-Ser-Leu-Glu-Asp-Ile-Leu-His-Gln-Val-Glu-Asn-Lys. Erfindungsgemäß werden durch Immunisieren mit synthetischen Peptiden im tierischen System Antikörper induziert und gegebenenfalls durch Immunadsorption gereinigt. Diese Antikörper reagieren spezifisch mit den zur Immunisierung und Reinigung verwendeten Peptiden.

Durch Auswahl entsprechender Peptide als Immunadsorbentien können Antikörper selektioniert werden, die bevorzugt spezifisch mit der antigenen Determinante des Fragments E, die der Plasmin-Spaltstelle dieses Moleküls entsprechen, reagieren. In dem bevorzugten Fall, daß Peptide, die Sequenzen aus der C-terminalen Region der Plasmin-Erkennungssequenz aufweisen, sowohl zur Immunisierung als auch zur immunadsorptiven Reinigung verwendet werden, werden Antikörper gegen diese Sequenzen angereichert.

Nach den, dem Fachmann an sich bekannten Verfahren können vorteilhafterweise auch monoklonale Antikörper mit den erfindungsgemäßen Eigenschaften hergestellt werden.

Die erfindungsgemäß gewonnenen Antikörper können für dem Fachmann an sich bekannte, homogene und heterogene Immunoassays, wie z. B. Enzymimmunoassays oder freie oder Partikel-verstärkte Agglutinationsreaktionen eingesetzt werden. Bevorzugterweise werden sie dazu an einen festen Träger gekoppelt. Als feste, wasserunlösliche Träger sind dem Fachmann an sich bekannte, natürliche und synthetische, organisch und anorganische Polymere geeignet, wie beispielsweise: Polystyrol, Polydextrane, Polypropylen, Polyvinylchlorid, Polyvinylidenfluorid, Polyacrylamid, Agarose, Latex, Magnetit, poröses Glaspulver, Erythrozyten, Leukozyten, Blutplättchen oder Copolymere aus Styrol-Butadien, Styrol-Methacrylsäure oder Methacrylat-Methacrylsäure. Als geeignete geometrische Ausführungsformen kommen Röhren, Kugeln oder Mikrotitrationsplatten in Betracht.

In einer bevorzugten Weise erfolgt die erfindungsgemäße Bestimmung des Gehaltes an Fragment E durch Inkubation der Probe mit an partikulären Trägern immobilisierten Antikörpern, wobei die Konzentration des durch die immobilisierten Antikörper gebundenen Fragments E durch die dabei entstehende Trubung turbidimetrisch oder nephelometrisch detektiert wird.

Erfindungsgemäß kann mit einem derart immobilisierten Antikörper auch die Konzentration des D-Dimer/E-Komplexes bestimmt werden. Voraussetzung ist die Verwendung eines spezifischen Antikörpers gegen D-Dimer als Zweitantikörper, der entweder an den gleichen oder an eigenen Partikeln immobilisiert ist. Einer der beiden Antikörper kann auch frei in der Lösung vorliegen, so daß sich Immunkomplexe der Form: Partikel-Antikörper 1/Antigen/freier Antikörper 2/Antigen/ Partikel-Antikörper 1 bilden und nephelometrisch oder turbidimetrisch quantifiziert werden.

Desweiteren sind bevorzugt heterogene Nachweismethoden, wobei erfindungsgemaße Antikörper in der ELISA-Technik an der Festphase immobilisiert sind. In einem ersten Inkubationsschritt erfolgt die Bindung des Fragments E oder des D-Dimer/E Komplexes an den immobilisierten Antikörper. In einem zweiten Inkubationsschritt mit dem gleichen oder einem anderen Antikörper wird das gebundene Antigen detektiert. Dieser zweite Antikörper muß eine Eigenschaft aufweisen, die meßbar ist, z. B. die Fähigkeit zur Umsetzung oder Bindung eines chromogenen Substrates.

Der zweite Antikörper kann z. B. mit einem Enzym, einem fluoreszierenden Molekül, wie z. B. Fluoresceinisothiocyanat, einer radioaktiven Markierung oder einem zur Chemolumineszenz fähigen Molekül versehen werden. Bevorzugterweise wird dieser zweite Antikörper mit einem Markerenzym gekoppelt, besonders bevorzugt ist Peroxidase.

Die Bestimmung von Fragment E bzw. D-Dimer/E-Komplex kann auch durch gleichzeitige Inkubation der Probe, bevorzugt von Plasma und markiertem Antikörper mit den immobilisierten Antikörpern erfolgen. Darüber hinaus ist ein kompetitives Bestimmungsverfahren möglich, bei dem markiertes und unmarkiertes Fragment E bzw. D-Dimer/E-Komplex um die Bindungsstelle der immobilisierten Antikörper konkurrieren. Der auf diese Weise bestimmte Gehalt an Fragment E bzw. D-Dimer/E-Komplex läßt eine Aussage über den Aktivierungsgrad des fibrinolytischen Systems zu.

Besonders bevorzugt sind die in den Beispielen angegebenen Ausführungsformen. Die Beispiele erläutern die Erfindung, schranken sie aber in keiner Weise ein.

Es werden folgende Abkürzungen verwendet:
- ELISA: Enzymimmunoassay (enzyme linked immunosorbent assay)
- KLH: Keyhole Limpet Hemocyanin (Haemocyanin einer marinen Napfschnecke)
- PBS: Phospatgepufferte Kochsalzlösung (phosphate buffered saline)
- Tris: Tris-(hydroxymethyl)-aminomethan
- OD: Extinktion (optical density)
- Cys: Cystein
Aminosäuren können in der D- oder L-Konfiguration vorliegen; sofern nicht extra vermerkt, liegen sie in der L-Form vor.
- Val: Valin
- Asp: Asparaginsäure
- Lys: Lysin
- Cys: Cystein
- Leu: Leucin
- Gln: Glutamin
- Ser: Serin
- Glu: Glutaminsäure
- Ile: Isoleucin
- His: Histidin
- Asn: Asparagin
- Boc: t-Butoxycarbonyl
- Fmoc: 9-Fluorenylmethoxycarbonyl
- Mtr: 4-Methoxy-2, 3, 6-trimethylphenylsulfonyl
- DMF: Dimethylformamid
- HOBt: Hydroxybenzotriazol
- DTT: Dithiothreitol
- Trt: Trityl

### Beispiel 1

### Herstellung eines Antigens zum Immunisieren

a) Synthese von Cys-Val-Asp-Lys-Asp-Leu-Gln-Ser-Leu-Glu--Asp-Ile-Leu-His-Gln-Val-Glu-Asn-Lys
   1 g Fmoc-Lys (Boc)-p-alkoxybenzylesterharz wurde 2 x mit 15 ml DMF 1 min gewaschen und die Fmoc Gruppe mit 15 ml 20 % Piperidin/ DMF (v/v) (1 x 3 min, 1 x 10 min) abgespalten. Das Harz wurde daraufhin je 3 x mit DMF bzw. Isopropanol (je 15 ml) und 2 x mit 15 ml DMF gewaschen. Es wurden 1,5 mMol Fmoc-Aminosäure und 2,25 mMol HOBt in 15 ml DMF gelöst zum Harz gegeben und nach Zusatz von 1,65 ml einer 1 M Diisopropylcarbodiimid-Lösung in Dichlormethan 1,5 h bei Raumtemperatur geschüttelt. Die Reaktion wurde mit einem Ninhydrintest auf Vollständigkeit geprüft. Danach wurde das Harz je 3 x mit DMF bzw. Isopropanol (je 15 ml) gewaschen und ein neuer Zyklus begonnen. Als letzte Aminosäure wurde eine Boc-Cys (Trt) benutzt. Das Harz wurde 3 x mit je 15 ml Isopropanol und Diethylether gewaschen und im Hochvakuum getrocknet. 1,9 g Harz wurden mit 1 ml Thioanisol, 1 ml Ethandithiol und 18 ml Trifluoressigsäure 2 h bei Raumtemperatur gerührt, abfiltriert, das Harz mit 3 Portionen Trifluoressigsäure/Dichlormethan (1:1) gewaschen und die Filtrate in Ether kristallisiert. Das Rohpeptid wurde mit Diethylether gewaschen und getrocknet. Das Peptid wurde an ^{R}Sephadex G 25 in 0,5 % Essigsäure chromatographiert. Ausbeute: 480 mg. 100 mg dieses Produktes (=Immunisierungspeptid) wurden zur Weiterreinigung an einer präparativen HPLC-Anlage an reversed-phase Material chromatographiert (0.1 % Acetonitril, Gradientenbetrieb). Der Peptidpool wurde gefriergetrocknet. Ausbeute: 38 mg.
   Nach dem gleichen Verfahren wurden folgende Teilsequenzen des Immunisierungspeptids zur Isolierung spezifischer Antikörper hergestellt:
   Reinigungspeptid 1: Cys-His-Gln-Val-Glu-Asn-Lys
   Reinigungspeptid 2: Cys-Asp-Ile-Leu-His-Gln-Val
   Reinigungspeptid 3: Cys-Ser-Leu-Glu-Asp-Ile-Leu
   Reinigungspeptid 4: Cys-Asp-Leu-Gln-Ser-Leu-Glu
   Reinigungspeptid 5: Cys-Val-Asp-Lys-Asp-Leu-Gln
b) Konjugatherstellung
   20 mg KLH wurden in 0,05 M Natriumphosphatpuffer pH 8,0 gelöst und mit 2 mg -Maleimidobuttersäurehydroxysuccinimidester 1 h lang gerührt. Das Protein wurde an einer ^{R}Sephadex G 50 (2 x 30 cm) (0,1 M Natriumphosphat, 0,5 mM EDTA, pH 6,0) chromatographiert. Das Eluat wurde auf 5 ml konzentriert und mit 20 mg Immunisierungspeptid 1 inkubiert. Nach Dialyse und Lyophilisation wurden 32 mg Immunisierungspeptidkonjugat erhalten.

### Beispiel 2

### Immunisieren von Kaninchen

5 Kaninchen wurden mit jeweils 2,6 mg Antigen pro Tier über einen Zeitraum von 8 Wochen immunisiert. Die Applikationen des Peptid-KLH-Konjugats erfolgten subkutan in die Nähe von Lymphknoten. Nach 3 Entnahmen wurden die Tiere entblutet und die Rohantiseren mit Konservierungsmittel stabilisiert. Ausbeute: ca. 200 ml Antiserum pro Tier.

### Beispiel 3

### Herstellen von Immunadsorbentien

Für die affinitätschromatographische Reinigung der Rohantiseren wurden je 25 mg der Reinigungspeptide 1 bis 5 (wie in Beispiel la hergestellt), die Teile des Immunisierungspeptids umfassen, an einer Festphase kovalent immobilisiert. Die Kopplungsreaktion erfolgte mit je 5 g Bromcyan-aktivierter Sepharose nach einem beschriebenen Verfahren (Axen, R. et al., Nature, 214:1302 (1967)). Anschließend wurde das Immunadsorbens jeweils mit phosphatgepufferter Kochsalzlösung (PBS; 0,15 mol/L, pH 7,2) und Essigsäure (0,5 ml/l, pH 2,5) gewaschen. Vor der Verwendung wurde das Adsorbens mit dem 3-fachen Gelvolumen PBS äquilibriert. Ausbeute: je ca. 20 ml Peptid-Sepharose.

### Beispiel 4

### Isolieren spezifischer Antikörper

Je 50 ml Rohantiserum von Kaninchen, die mit dem Immunisierungspeptid gemäß Beispiel 2 immunisiert worden waren, wurden auf die mit PBS äquilibrierten 20 ml Peptid-Sepharosen aus Beispiel 3 (Durchmesser 1,6 cm; Höhe 9 cm) aufgetragen und anschließend mit PBS gewaschen, bis die Extinktion bei 280 nm ≤0,01 betrug. Danach erfolgten Waschschritte mit Kochsalzlösung (1 mol/L, pH 7,0) und entionisiertem Wasser (pH 7,0), wobei jeweils das 3-fache Gelvolumen verwendet wurde. Die Elution der Antikörper vom Immunadsorbens erfolgte mit einer Glycinlösung (0,2 mol/L, pH 2,5), die Antikörperlösung wurde mit einer gesättigten Trislösung auf pH 7,0 eingestellt und gegen PBS dialysiert. Die Antikörper wurden bis zur weiteren Verwendung bei -70 °C gelagert. Die Ausbeute war abhängig vom verwendeten Reinigungspeptid (RP) (Tabelle 1).

Tabelle 1: Ausbeute an Antikörpern aus 50 ml Antiserum gegen das Immunisierungspeptid gemäß Beispiel 1, nach Immunadsorption an Teilsequenzen des Immunisierungspeptids (RP1 - RP5).

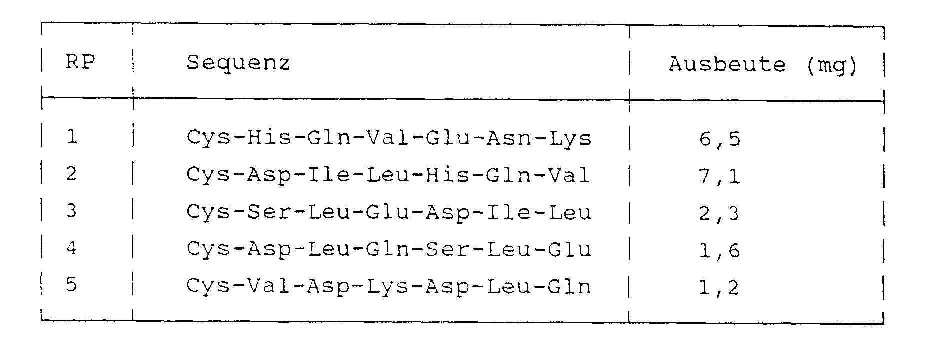

### Beispiel 5

### Herstellen von Fragment E aus Fibrinogen und Fibrin

a) Herstellung von Fibrin-E: Fragmente E1 und E2
   Die Herstellung der verschiedenen Abbaustufen des Fragments E (E1, E2, E3) erfolgte nach einem beschriebenen Verfahren (Olexa, S. & Budzynski, A.Z., "Binding phenomena of isolated unique plasmic degradation products of human cross-linked fibrin." J. Biol. Chem. 254: 4925-4932 (1979)). 250 mg Testfibrinogen (Behringwerke AG) wurden in 100 ml 0,05 mol/L Tris, 0,1 M NaCl (pH 7,8) gelöst und durch Zugabe von 250 Einheiten Thrombin, 225 Einheiten F XIII in Gegenwart von 10 mM CaCl₂ bei +37 °C zum Clotten gebracht. Der Fibrinclot wurde anschließend in 0,15 mol/L Tris-HCl (pH 7,4) gewaschen und in Gegenwart von 5 mM CaCl₂ durch Zugabe von 1 CTA Plasmin pro g Fibrin(ogen) über Nacht bei +37 °C gespalten. Die Reaktion wurde durch Zugabe von Aprotinin (100 KIU pro CTA Plasmin) gestoppt und die Spaltprodukte mittels Gelchromatographie auf Aca 34 Ultrogel (Saulendurchmesser 1,7 cm; Höhe 90 cm) getrennt. Als Laufpuffer diente 0,05 mol/L Tris, 0,1 mol/L NaCl, 0,028 mol/L Na-Citrat, 25 KIU/ml Antagosan mit pH 7,4. Ausbeute: ca. 100 mg DD/E-Komplex. Der DD/E-Komplex (Komplex aus D-Dimer mit den Fragmenten E1 und E2) wurde anschließend in 0,05 mol/L Citrat, 3 M Harnstoff bei pH 5,5 bei +37 °C dissoziiert und durch Rechromatographie auf Aca 34 Ultrogel die Fragmente E1 und E2 von D-Dimer getrennt (Verhältnis von E1 zu E2 ca. 2:1). Als Laufpuffer diente 0,05 mol/L Tris, 1,0 mol/L NaCl, 0,028 mol/L Na-Citrat mit pH 7,4. Ausbeute: ca. 25 mg El/E2-Gemisch.
b) Herstellung von Fibrinogen-E: Fragment E3
   250 mg Testfibrinogen (Behringwerke AG) wurden in 100 ml 0,05 mol/L Tris, 0,1 M NaCl (pH 7,8) gelöst und nach Zugabe von 4,7 CTA Plasmin in Gegenwart von 5 mM CaCl₂ über 30 min bei +37 °C gespalten. Die Reaktion wurde durch Zugabe von Aprotinin (20000 KIU) abgestoppt. Die Trennung der Spaltprodukte erfolgte mittels Gelchromatographie auf Aca 34 Ultrogel (Säulendurchmesser 4 cm; Höhe 90 cm). Als Laufpuffer diente 0,05 mol/L Tris, 1,0 mol/L NaCl, 0,028 mol/L Na-Citrat mit pH 5,7. Ausbeute: ca. 32 mg Fragment E3.

### Beispiel 6

### Erzeugung von löslichem Fibrin in humanem Plasma

20 ml eines humanen Plasmapools wurden mit 0,6 IU Thrombin versetzt und für 90 min bei +37 °C inkubiert. Die Reaktion wurde durch Zugabe von 6 ATU Hirudin (Fa. Hoechst AG) abgestoppt. Die Bestimmung der Fibrinmonomere erfolgte mittels tPA-Stimulationstest (Fa. Kabi, Schweden). Höhere Konzentrationen an Fibrinmonomeren führte bereits zur Aggregation, so daß im Plasma keine höheren Werte zu erwarten sind. Ausbeute: 65 µg Fibrinmonomere/ml Plasma.

### Beispiel 7

### Verwendung der erfindungsgemäßen Antikörper im ELISA

a) Herstellen von Antikörper-beschichteten Mikrotiterplatten
   Die in Beispiel 4 gewonnenen Antikörper wurden mit einer Natriumphosphatlösung (0,01 mol/L, pH 5,5) auf eine Konzentration von 5 µg/ml verdünnt und durch Adsorption an Mikrotiterplatten (Typ B, Fa. Nunc, Dänemark) immobilisiert. Pro Vertiefung wurden 100 µl Antikörperlösung für 20 h bei 20 °C inkubiert, anschließend die Flüssigkeit abgesaugt und mit dem Natriumphosphatpuffer 3-mal gewaschen. Danach wurden 100 µl einer Rinderserumalbuminlösung (0,1 g/L in Natriumphosphat 0.01 mol/L, pH 5,5) in jede Vertiefung eingefüllt und 1 h bei 20 °C inkubiert. Nach 3-maligem Waschen mit der Natriumphosphatlösung wurden die Mikrotiterplatten luftdicht verschlossen bei +4 °C gelagert.
b) Durchführung des Enzymimmunoassays
   Die zu testenden Proben wurden mit Inkubationspuffer (PBS, 0.05 % Tween 20, pH 7.2) 1:2 verdünnt und jeweils 100 µl pro Vertiefung der beschichteten Mikrotiterplatten (nach Beispiel 5 a) 60 min bei +37 °C inkubiert. Anschließend wurde die Inkubationslösung entfernt und die Vertiefungen 3 x mit je ca. 300 µl Waschlösung (0,02 mol/L Natriumphosphat, 0,05 % Tween 20, pH 7,6) gewaschen. Anschließend wurden 100 µl Peroxidase-konjugierte anti-Fragment E Antikörper (Fa. Behringwerke AG, Marburg, FRG) zugefügt und die Mikrotiterplatte 60 min bei +37 °C inkubiert. Nach Entfernen der Konjugat-Lösung und 2-maligem Waschen wurden 100 µl Substrat-Chromogen-Lösung (Wasserstoffperoxid, o-Phenylendiamin) zugefügt und die Mikrotiterplatte bei Raumtemperatur inkubiert. Nach 30-minütiger Inkubation wurde die Peroxidase mit Schwefelsäure inaktiviert und die Extinktion der Reaktionslösung bei 490 nm bestimmt.

### Beispiel 8

### Untersuchung der immunadsorptiv gereinigten Antikörper

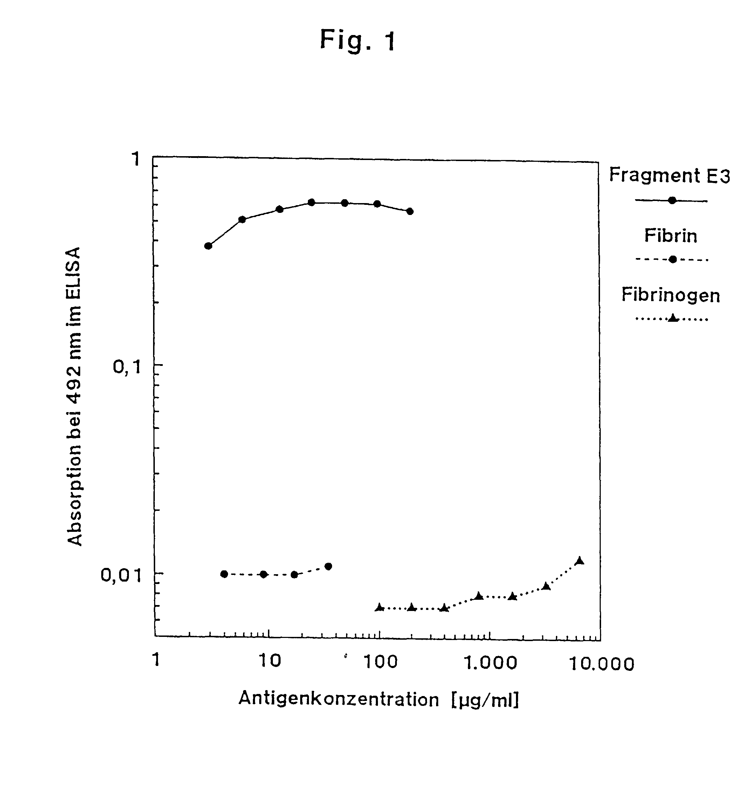

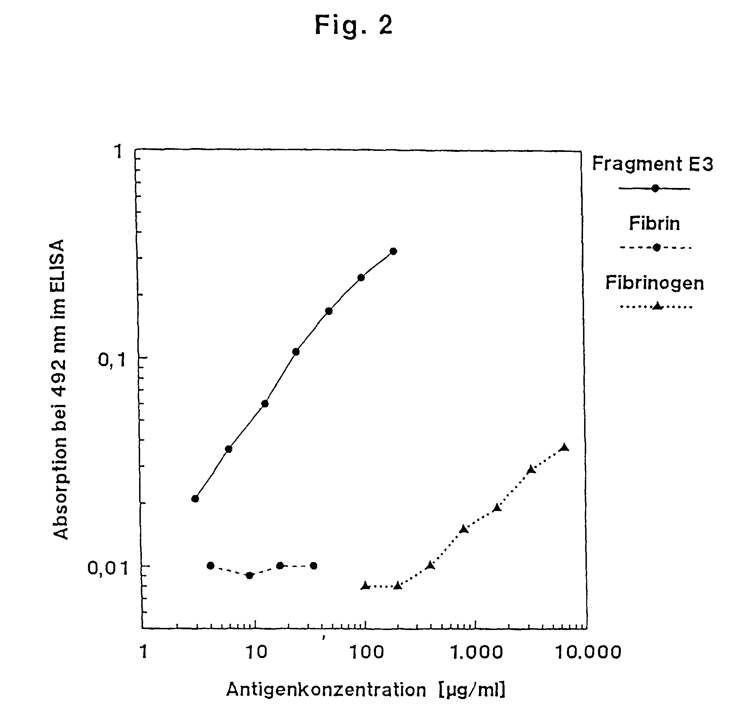

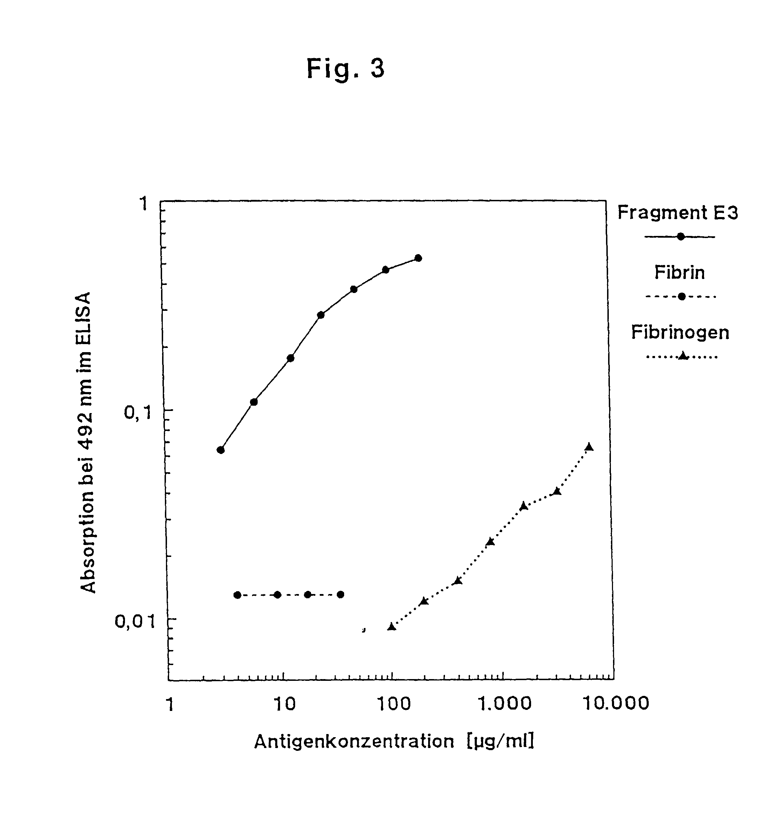

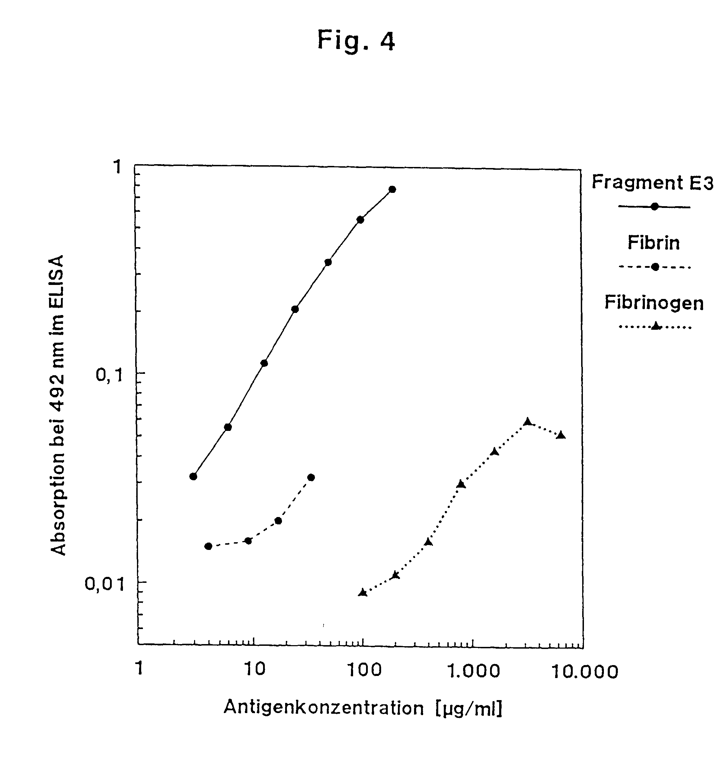

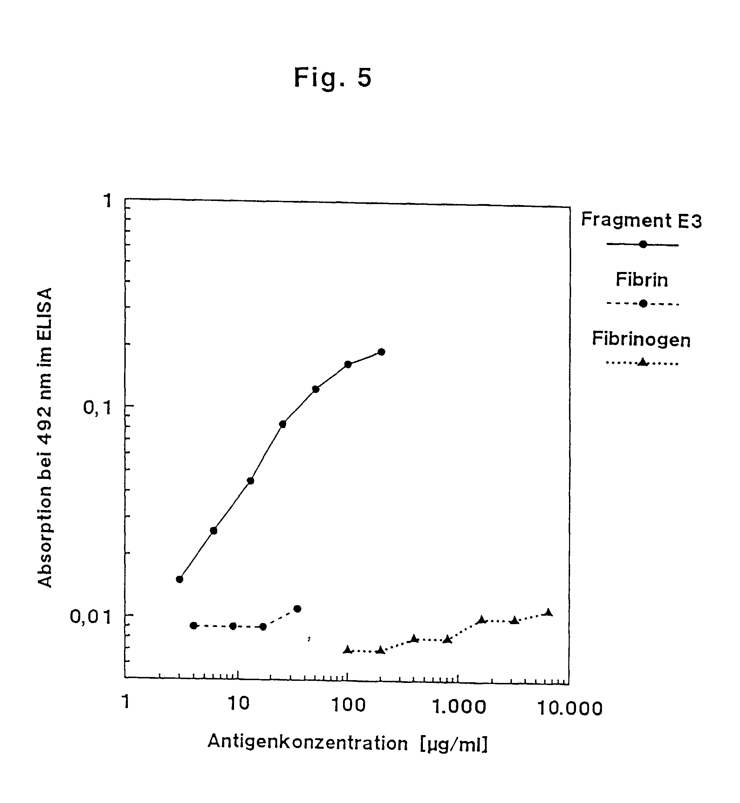

a) Reaktion mit Fibrinogen, Fibrin und Fragment E3
Zur Untersuchung der Spezifität der nach Beispiel 4 mit den Reinigungspeptiden 1 bis 5 (RP1-RP5) gewonnenen Antikörper wurde gemäß Beispiel 7 im ELISA die Reaktivität mit Fibrinogen, Fibrin (dem Abbauprodukt des Fibrinogens durch Thrombineinwirkung) und mit Fragment E3 (dem Abbauprodukt des Fibrinogens durch Plasmineinwirkung) vergleichend untersucht. Als Fibrinquelle wurde nach Beispiel 6 in Plasma erzeugtes Fibrinmonomer mit einer Konzentration von 34 µg/ml verwendet, als Fragment E3, E3 erzeugt nach Beispiel 5b) mit 200 µg/ml und Testfibrinogen (Fa. Behringwerke AG) mit 6400 µg/ml. Die Proben wurden seriell 1:2 in Inkubationspuffer verdünnt (Beispiel 7).
Aus den Ergebnissen (siehe Tabelle 2 und Figuren 1 bis 5) ist ersichtlich, daß die erfindungsgemäß hergestellten Antikörper sehr stark mit dem durch Plasmineinwirkung entstandenen Fibrinogenspaltprodukt reagieren. Die über RP1 oder RP5 immunadsorptiv gereinigten Antikörper zeigen keinerlei Kreuzreaktion mit Fibrinmonomer oder Fibrinogen. Auch eine Reinigung der Antikörper über RP2, RP3 und RP4 liefert Antikörper, die nicht oder vernachlässigbar mit Fibrinmonomeren reagieren. Die Reaktion mit Fibrinogen ist bei RP3-isolierten Antikörper ebenfalls vernachlässigbar. Somit ist dieses Verfahren geeignet, spezifische Antikörper zu erhalten, die keine weitere "Nachreinigung" benötigen.
Tabelle 2: Reaktion der erfindungsgemäß isolierten Antikörper gegen Teilsequenzen des Immunisierungspeptids mit Fibrinogen und den durch Thrombin-(Fibrinmonomere) oder Plasminverdau (Fragment E3) entstandenen Spaltprodukten in Puffer im ELISA. Angegeben sind die Meßwerte im ELISA; Antigenkonzentrationen in µg/ml.

**Tabelle 2**

| RP | Fibrinogen | | Fragment E3 | | Fibrin | |
|---|---|---|---|---|---|---|
| | Konz. | Ext. | Konz. | Ext. | Konz. | Ext. |
| 1 | 0 | 0,007 | 0 | 0,007 | | |
| | 100 | 0,007 | 3 | 0,377 | 4 | 0,010 |
| | 200 | 0,007 | 6 | 0,512 | 9 | 0,010 |
| | 400 | 0,007 | 13 | 0,576 | 17 | 0,010 |
| | 800 | 0,008 | 25 | 0,624 | 34 | 0,011 |
| | 1600 | 0,008 | 50 | 0,624 | | |
| | 3200 | 0,009 | 100 | 0,620 | | |
| | 6400 | 0,012 | 200 | 0,574 | | |
| 2 | 0 | 0,007 | 0 | 0,007 | | |
| | 100 | 0,008 | 3 | 0,021 | 4 | 0,010 |
| | 200 | 0,008 | 6 | 0,036 | 9 | 0,009 |
| | 400 | 0,010 | 13 | 0,060 | 17 | 0,010 |
| | 800 | 0,015 | 25 | 0,107 | 34 | 0,010 |
| | 1600 | 0,019 | 50 | 0,167 | | |
| | 3200 | 0,029 | 100 | 0,243 | | |
| | 6400 | 0,037 | 200 | 0,325 | | |
| 3 | 0 | 0,007 | 0 | 0,011 | | |
| | 100 | 0,009 | 3 | 0,064 | 4 | 0,013 |
| | 200 | 0,012 | 6 | 0,109 | 9 | 0,013 |
| | 400 | 0,015 | 13 | 0,176 | 17 | 0,013 |
| | 800 | 0,023 | 25 | 0,282 | 34 | 0,013 |
| | 1600 | 0,034 | 50 | 0,375 | | |
| | 3200 | 0,040 | 100 | 0,464 | | |
| | 6400 | 0,065 | 200 | 0,529 | | |
| 4 | 0 | 0,007 | 0 | 0,007 | | |
| | 100 | 0,009 | 3 | 0,032 | 4 | 0,015 |
| | 200 | 0,011 | 6 | 0,055 | 9 | 0,016 |
| | 400 | 0,016 | 13 | 0,113 | 17 | 0,020 |
| | 800 | 0,030 | 25 | 0,207 | 34 | 0,032 |
| | 1600 | 0,043 | 50 | 0,349 | | |
| | 3200 | 0,060 | 100 | 0,558 | | |
| | 6400 | 0,052 | 200 | 0,781 | | |
| 5 | 0 | 0,008 | 0 | 0,007 | | |
| | 100 | 0,007 | 3 | 0,015 | 4 | 0,0091 |
| | 200 | 0,007 | 6 | 0,026 | 9 | 0,0091 |
| | 400 | 0,008 | 13 | 0,045 | 17 | 0,009 |
| | 800 | 0,008 | 25 | 0,084 | 34 | 0,011 |
| | 1600 | 0,007 | 50 | 0,124 | | |
| | 3200 | 0,007 | 100 | 0,165 | | |
| | 6400 | 0,008 | 200 | 0,191 | | |

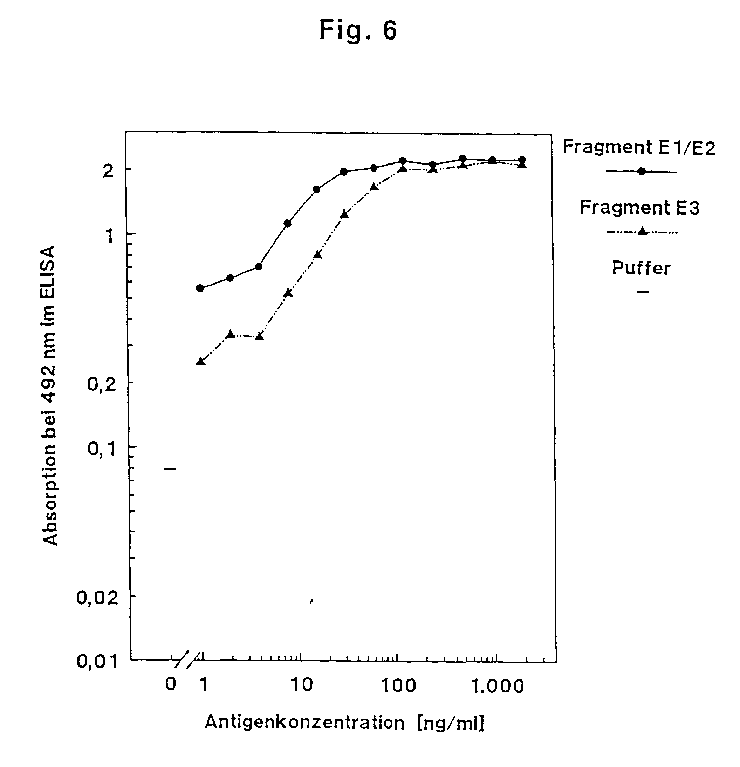

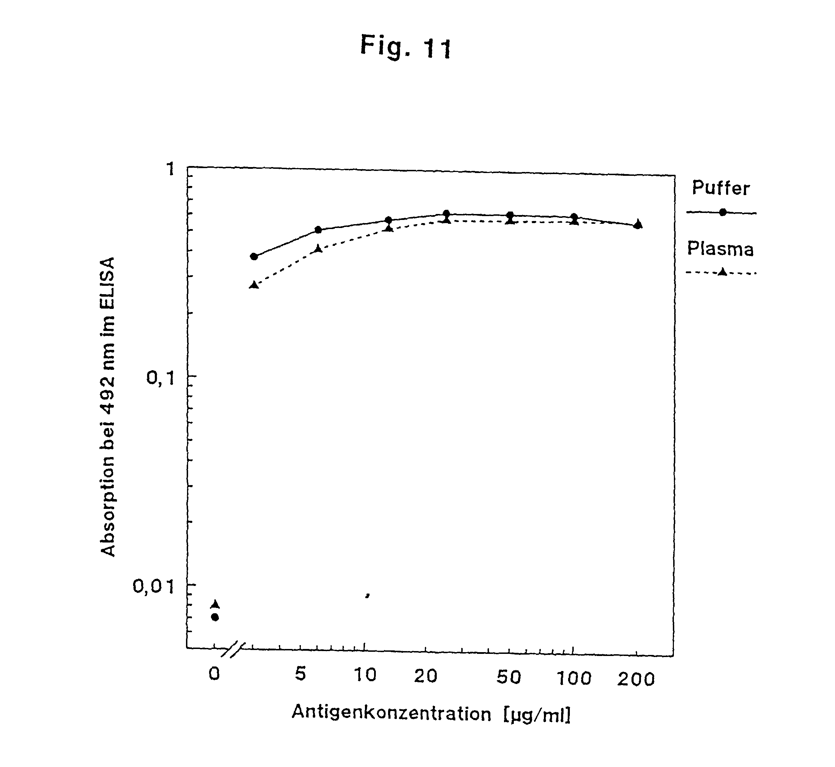

b) Reaktion mit verschiedenen Fragmenten E
Analog zu Beispiel 8a) wurde die Reaktivität der über verschiedene Teil sequenzen des Immunisierungspeptids immunadsorptiv gereinigten Antikörper mit dem Fragment E aus quervernetztem Fibrin (Fragment-Gemisch E1/E2; hergestellt gemäß Beispiel 5a) und aus Fibrinogen (Fragment E3; hergestellt gemäß Beispiel 5b) vergleichend untersucht. Die Konzentration der Stammlösungen betrug 2 µg/ml.
Aus Tabelle 3 (siehe auch Figuren 6 - 10) ist ersichtlich, daß alle erfindungsgemäß hergestellten Antikörper sowohl mit dem Fragment E3, als auch mit Fragment E1/2-Gemisch gleich gut reagieren. Antikörper, gereinigt über RP4 und RP5, diskriminieren hingegen zwischen Fragment E3 aus Fibrinogen und E1/2 aus quervernetztem Fibrin. Die Nachweisgrenze lag in diesem Beispiel im Bereich von 1 ng/ml. Die erfindungsgemäßen Antikörper sind somit zur sensitiven Diagnose sowohl der primären, als auch der sekundären Fibrinolyse geeignet.
Tabelle 3: Reaktion der erfindungsgemäß isolierten Antikörper gegen Teilsequenzen des Immunisierungspeptids mit Fragment E (E3) aus Fibrinogen (primäre Fibrinolyse) und mit Fragment E (E1/E2) aus quervernetztem Fibrin (sekundäre Fibrinolyse) in Puffer im ELISA. Angegeben sind die Meßwerte im ELISA; Antigenkonzentrationen in ng/ml. 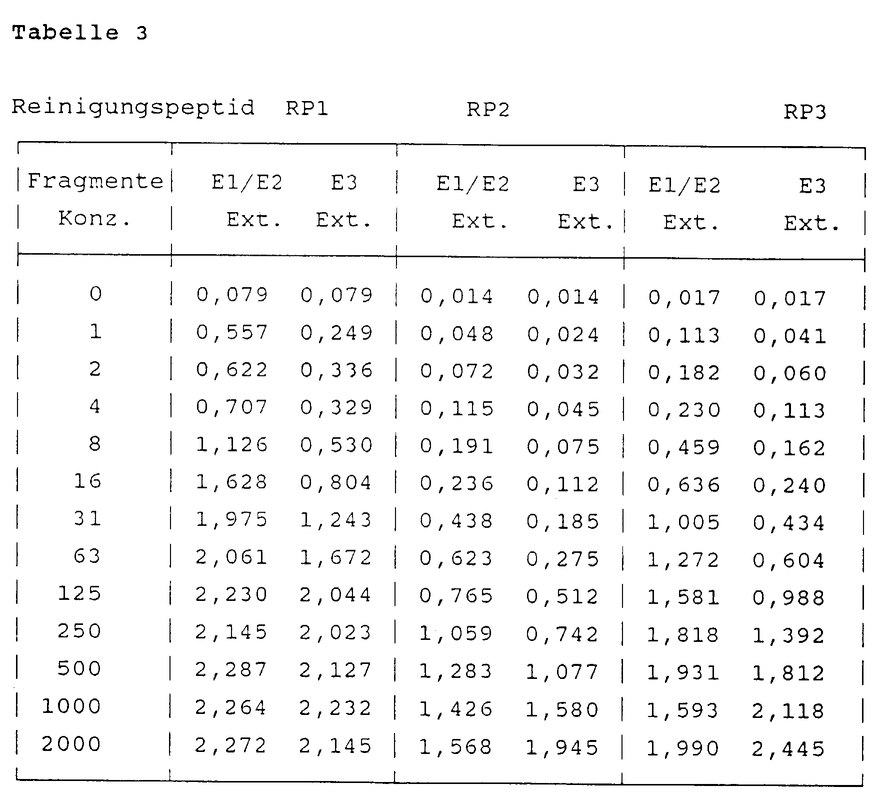 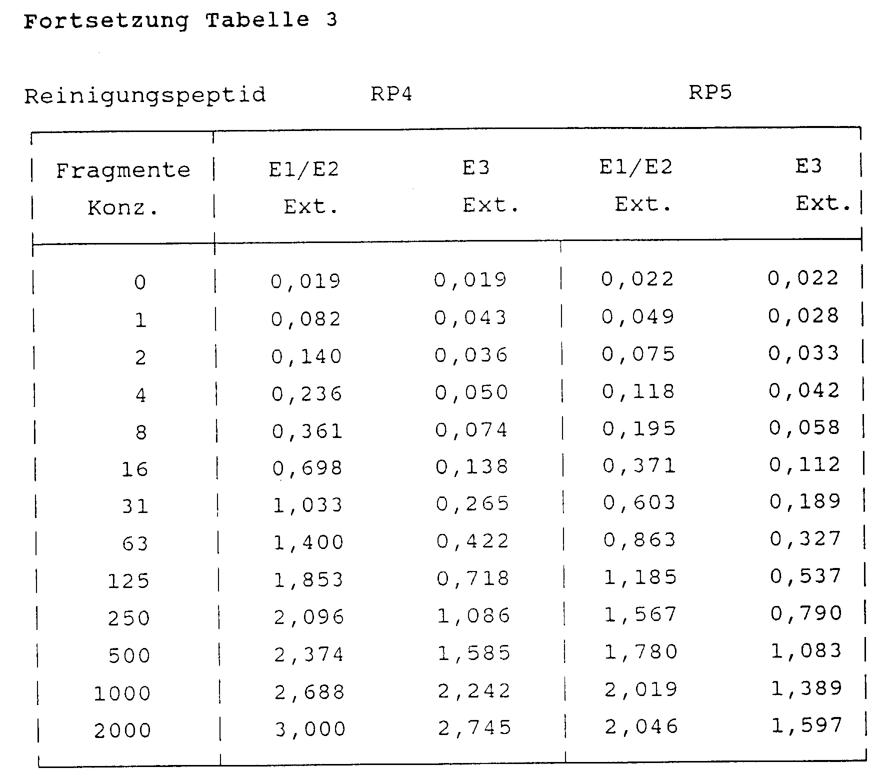
c) Reaktion mit Fragment E in Puffer und Plasma
Zur Überprüfung, ob die erfindungsgemäß gereinigten Antikörper (siehe Beispiel 4) auch dafür geeignet sind, Fibrin(ogen)spaltprodukte im Plasma nachzuweisen, wurde analog zu Beispiel 8a) die Reaktivität der gereinigten Antikörper mit dem Fragment E aus Fibrinogen (Fragment E3; hergestellt gemäß Beispiel 5b) im Puffersystem und nach Versetzen eines Humanplasmapools mit gereinigtem Fragment E3 vergleichend untersucht. Die Konzentration der Stammlösungen betrug 200 µg/ml.
Aus Tabelle 4 (siehe auch Figuren 11 - 13) ist ersichtlich, daß auch in Gegenwart des nativen Fibrinogens im Plasma Fibrin(ogen)spaltprodukte nachgewiesen werden können. Die Meßsignale im Plasma sind auf Grund der unterschiedlichen Matrix nur geringfügig niedriger.
Tabelle 4: Reaktion der erfindungsgemäß isolierten Antikörper gegen Teilsequenzen des Immunisierungspeptids (RP1 bis RP3) mit Fragment E (E3) in Puffer und Plasma im ELISA. Angegeben sind die Meßwerte im ELISA; Antigenkonzentrationen in µg/ml.

**Tabelle 4**

| Reinigungspeptid | RP1 | | RP2 | | RP3 | |
|---|---|---|---|---|---|---|
| E3 Konz. | Puffer Ext. | Plasma Ext. | Puffer Ext. | Plasma Ext. | Puffer Ext. | Plasma Ext. |
| 0 | 0,007 | 0,008 | 0,007 | 0,010 | 0,011 | 0,011 |
| 3 | 0,377 | 0,273 | 0,021 | 0,018 | 0,064 | 0,047 |
| 6 | 0,512 | 0,414 | 0,036 | 0,027 | 0,109 | 0,075 |
| 13 | 0,576 | 0,525 | 0,060 | 0,049 | 0,176 | 0,132 |
| 25 | 0,624 | 0,581 | 0,107 | 0,075 | 0,282 | 0,190 |
| 50 | 0,624 | 0,588 | 0,167 | 0,120 | 0,375 | 0,270 |
| 100 | 0,620 | 0,588 | 0,243 | 0,191 | 0,464 | 0,342 |
| 200 | 0,574 | 0,580 | 0,325 | 0,279 | 0,529 | 0,435 |

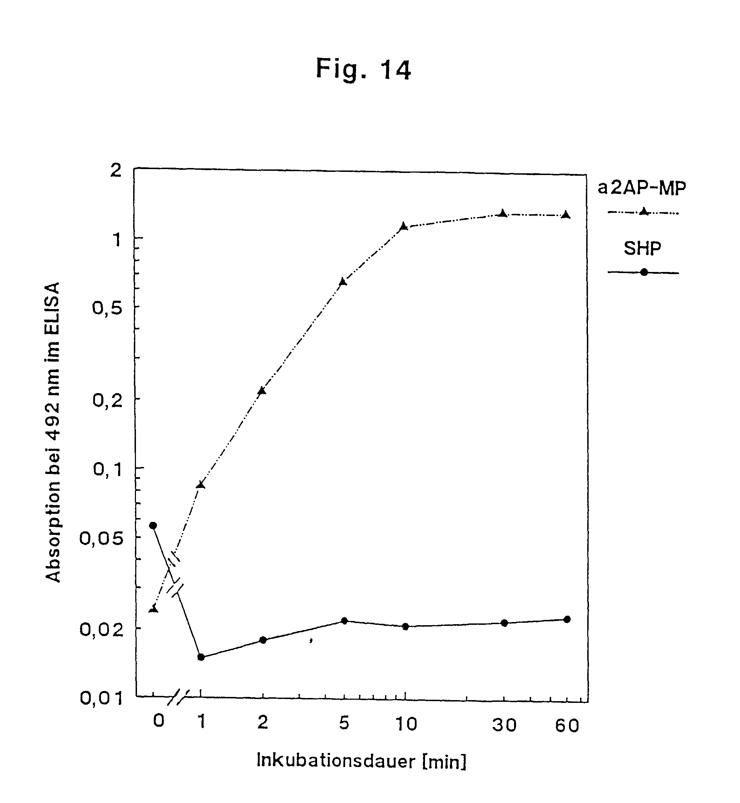

d) Reaktion mit in vitro erzeugten Fibrinspaltprodukten
3 ml eines humanen Plasmapools bzw. eines α2-Antiplasmin Mangelplasmas wurden jeweils mit 30 ATU Hirudin (Fa. Hoechst AG) versetzt und anschließend bei +37 °C mit Plasmin (Endkonzentration 1 CTA/ml) inkubiert. Nach 0, 1, 2, 5, 10, 30 und 60 min wurden jeweils 600 µl der Plasmen mit 60 µl Antagosanlösung (Fa. Behringwerke AG; 100 APE/ml) versetzt und im ELISA mit den verschieden gereinigten Antikörpern aus Beispiel 4 gemäß Beispiel 7 die Entstehung der Fibrinogenspaltprodukte verfolgt.
Die in Tabelle 5 (siehe Figuren 14 - 16) aufgeführten Meßwerte im ELISA zeigen, daß mit den gegen die Reinigungspeptide RP1 bis RP3 isolierten Antikörpern auf der Festphase die im α2-Antiplasmin Mangelplasma durch Plasminzugabe in vitro erzeugten Fibrinogenabbauprodukte nachgewiesen werden können. Im Plasmapool von normalen Blutspendern hingegen wird das zugegebene Plasmin durch a2-Antiplasmin inhibiert und es entstehen keine Fibrinogenspaltprodukte. Entsprechend ist im ELISA kein erhöhtes Meßsignal im Vergleich zum Zeitpunkt 0 min zu verzeichnen.
Tabelle 5: Zeitkinetik der Entstehung von Fibrinogenspaltprodukten bei der Behandlung von normalen (SHP) bzw. eines α2-Antiplasmin Mangelplasmas (α2-MP) mit Plasmin. Aufgeführt sind die Meßwerte bei Bestimmung der Fibrinogenspaltprodukte im ELISA nach Beispiel 7 mit den gegen die Teilsequenzen des Immunisierungspeptids RP1, RP2 oder RP3 gereinigten Antikörpern auf der Festphase.

**Tabelle 5**

| Reinigungspeptid | RP1 | | RP2 | | RP3 | |
|---|---|---|---|---|---|---|
| Zeit min | SHP Ext. | α2-MP Ext. | SHP Ext. | α2-MP Ext. | SHP Ext. | α-MP Ext. |
| 0 | 0,056 | 0,024 | 0,073 | 0,066 | 0,081 | 0,076 |
| 1 | 0,015 | 0,084 | 0,043 | 0,096 | 0,043 | 0,111 |
| 2 | 0,018 | 0,218 | 0,042 | 0,124 | 0,047 | 0,179 |
| 5 | 0,022 | 0,654 | 0,039 | 0,159 | 0,049 | 0,364 |
| 10 | 0,021 | 1,153 | 0,042 | 0,279 | 0,052 | 0,669 |
| 30 | 0,022 | 1,336 | 0,043 | 0,402 | 0,056 | 0,880 |
| 60 | 0,023 | 1,333 | 0,052 | 0,397 | 0,060 | 0,822 |

### Beispiel 9

### Verwendung der erfindungsgemaßen Antikörper im Agglutinationstest.

a) Herstellung eines Latexreagenz zur Bestimmung von Fibrin(ogen)spaltprodukten.
Die Herstellung von Latexreagenzien erfolgte nach Kapmeyer W.H. et al. "Automated nephelometric immuno-assays with novel shell/core particles." J. Clin. Lab. Anal. 2: 76-83 (1988). 1 ml eines Propfpolymerisats (4%ige Lösung; Fa. Behringwerke AG) wurde mit 0,1 ml einer Antikörper-Lösung (gereinigt gemäß Beispiel 4; Konzentration: 0,4 mg/ml; entspricht Kopplungsverhältnis 1:100) und 0,05 ml einer 20%igen wässrigen Tween^{R} 20 Lösung gemischt. Zur Aktivierung des Latex wurde die Lösung mit ca. 0,01 ml einer 1 N HCl-Lösung auf einen pH von 2 eingestellt. Nach einer 30-minütigen Inkubation bei Raumtemperatur wurden 0,25 ml einer gesättigten Natriumhydrogenphosphat-Lösung (pH 6,5) und 0,25 ml einer wässrigen Natriumcyanborhydrid-Lösung (25 mg/ml) hinzugefügt und intensiv gemischt. Die Kopplung des Antikörpers an die aktivierten Aldehydgruppen erfolgte über 1 h bei Raumtemperatur. Anschließend wurde das Latex-Antikörper Konjugat zentrifugiert (Beckman Zentrifuge, 40000xg, 30 min) und das Pellet in 1,5 ml eines 0,1 molaren Glycin-Puffers (pH 8,2; enthaltend 0,17 M NaCl und 0,5 % Tween^{R} 20) resuspendiert. Die Lösung wurde mit Ultraschall für ca. 5 s behandelt (Bronson Sonifier B 15). Diese Stammlösung wurde bei +4 °C gelagert.
b) Durchführung nephelometrischer Bestimmungen
Die Reaktion der anti-Fibrin(ogen)spaltprodukt Latices (anti-FSP-Latex) mit den Fibrin(ogen)spaltprodukten wurde am Nephelometer BNA, Fa. Behringwerke AG, Marburg, verfolgt. Die wie unter Beispiel 9 a) hergestellten Stammlösungen wurden mit physiologischer Kochsalzlösung auf eine Konzentration von 0,03 % verdünnt. 50 µl dieser Suspension wurden mit 20 µl Zusatzreagenz D-Dimer (Fa. Behringwerke AG) und 80 µl N-Diluens (Fa. Behringwerke AG) gemischt. Nach Zugabe von 50 µl Probe und 70 µl N-Diluens wurde die Trübungszunahme nach 12 minütiger Inkubation bestimmt.
c) Reaktion der Antikörper-beschichteten Latexpartikel mit Fibrin(ogen)spaltprodukten
Gegen Reinigungspeptid RP1 isolierte Antikörper gemäß Beispiel 4 wurden mit dem unter Beispiel 9 a) beschriebenen Verfahren an Latexpartikel mit einem Durchmesser von 230 nm gebunden. Die Reaktion des anti-FSP-Latex mit dem Fibrinogenspaltprodukt E3 und den Fibrinspaltprodukten E1/E2 wurde nach Beispiel 9 b) verfolgt. Die Antigene lagen in physiologischer Kochsalzlösung vor und wurden vom Gerät aus einer 2,5-fach konzentrierten Stammlösung entsprechend vor Messung verdünnt.
Die in Tabelle 6 aufgeführten Meßsignale zeigen eine von der Konzentration der Fibrin(ogen)spaltprodukte abhängige Steigerung der Trübungszunahme. Diese Agglutinationsreaktion beweist somit die Eignung der erfindungsgemäßen Antikörper zum Nachweis von Fibrin(ogen)spaltprodukten nach der Hypothese eines "internen Dimers" in den verschiedenen Fragmenten E.
Tabelle 6: Trübungszunahme bei Reaktion von anti-FSP-Latex mit Fibrin- und Fibrinogenspaltprodukten. Aufgeführt sind die Meßdifferenzen der Trübung (in gerätespezifischen Trübungseinheiten) die nach 12-minütiger Inkubation einer anti-FSP-Latex Suspension mit Fibrin(E1/E2) bzw. Fibrinogenspaltprodukten (E3) am Nephelometer (BNA; Behringwerke AG) erhalten wurden. Die Konzentration der Antigene ist in mg/l.

**Tabelle 6**

| Fragmente Konz. | E1/E2 Ext. | E3 Ext. |
|---|---|---|
| 1,3 | 602 | 414 |
| 2,5 | 876 | 459 |
| 5,0 | 1001 | 558 |
| 10,0 | 1288 | 699 |
| 20,0 | 1473 | 886 |

### Legende zu den Figuren:

### Figuren 1 - 5:

Reaktion von Antikörpern aus Kaninchen gegen Peptid 3 mit Fibrinogen, Fibrin und Fragment E3 im ELISA.

In Kaninchen wurden durch Immunisierung mit einem Peptid aus der carboxyterminalen Region der -Kette des Fragment E (Peptid 3) Antikörper erzeugt. Die Antikörper wurden immunadsorptiv an Festphasen-immobilisierten Hexapeptiden gereinigt, deren Sequenzen teilweise der des Immunisierungspeptides entsprach (Reinigungspeptid 1 bis RP 5). Diese Antikörper wurden zur Beschichtung von Mikrotitrationsplatten verwendet und die Reaktion mit Fibrinogen, Fibrin und dem Fragment E3 aus Plasmin-abgebauten Fibrinogen im Sandwich-ELISA getestet. Zur Detektion gebundenen Antigens wurde ein Konjugat aus unspezifischen Antikörpern gegen Fragment E und Meerrettich-Peroxidase verwendet. Dargestellt sind die Meßsignale im ELISA bei Verwendung von Fibrinogen und Fragment E3 in Probenpuffer, bzw. von Fibrin-haltigem Plasma.
- Fig. 1:: Antikörper, gereinigt gegen RP1.
- Fig. 2:: Antikörper, gereinigt gegen RP2.
- Fig. 3:: Antikörper, gereinigt gegen RP3.
- Fig. 4:: Antikörper, gereinigt gegen RP4.
- Fig. 5:: Antikörper, gereinigt gegen RP5.

### Figuren 6 - 10:

Reaktion von Antikörpern aus Kaninchen gegen Peptid 3 mit Fragment E aus Fibrinogen (E3) und aus Fibrin (E1/E2) im ELISA.

Ein Gemisch der Fragmente E1 und E2, die nach Plasmineinwirkung auf quervernetztes Fibrin aus dem D-Dimer/ E-Komplex isoliert wurden, sowie Fragment E3, das nach Plasmineinwirkung auf Fibrinogen gereinigt wurde, wurden im ELISA als Antigen eingesetzt. Verwendet wurden die gleichen Antikorper wie in der Legende zu den Abbildungen 1-5 beschrieben. Dargestellt sind die Meßsignale im ELISA bei Verwendung von Fragment E1/E2, Fragment E3, sowie das Hintergrundsignal bei Verwendung von Probenpuffer als Probe.
- Fig. 6:: Antikörper, gereinigt gegen RP1.

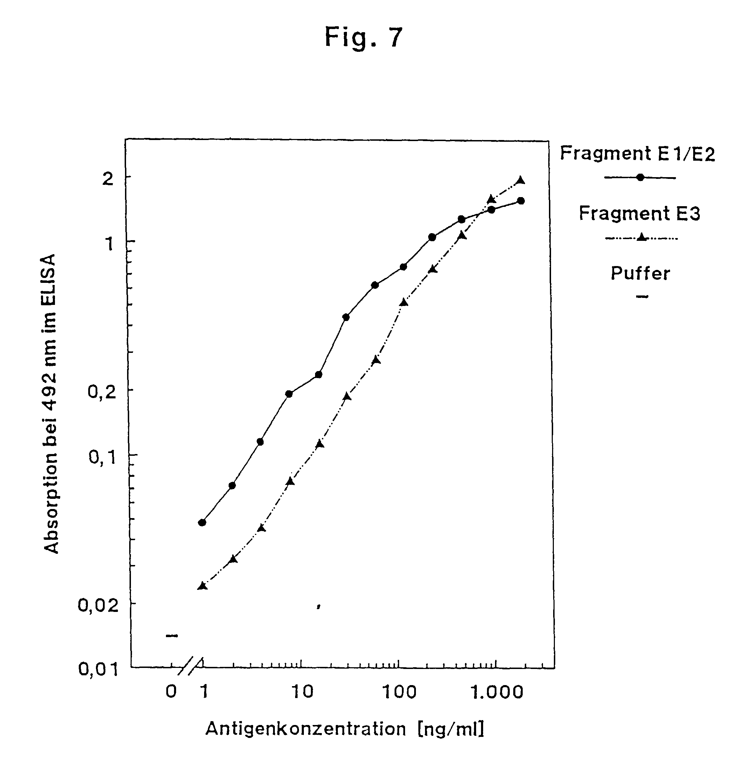

- Fig. 7:: Antikörper, gereinigt gegen RP2.

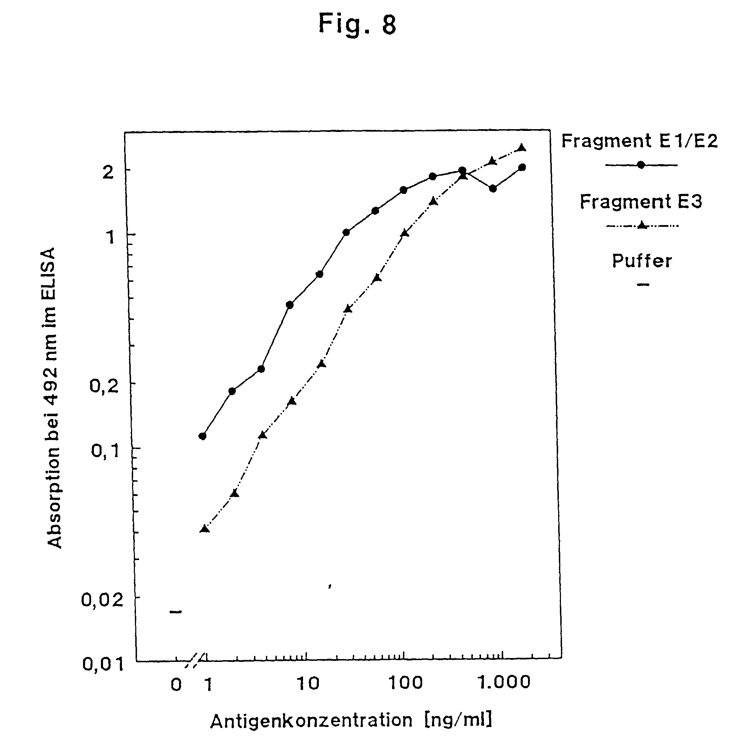

- Fig. 8:: Antikörper, gereinigt gegen RP3.

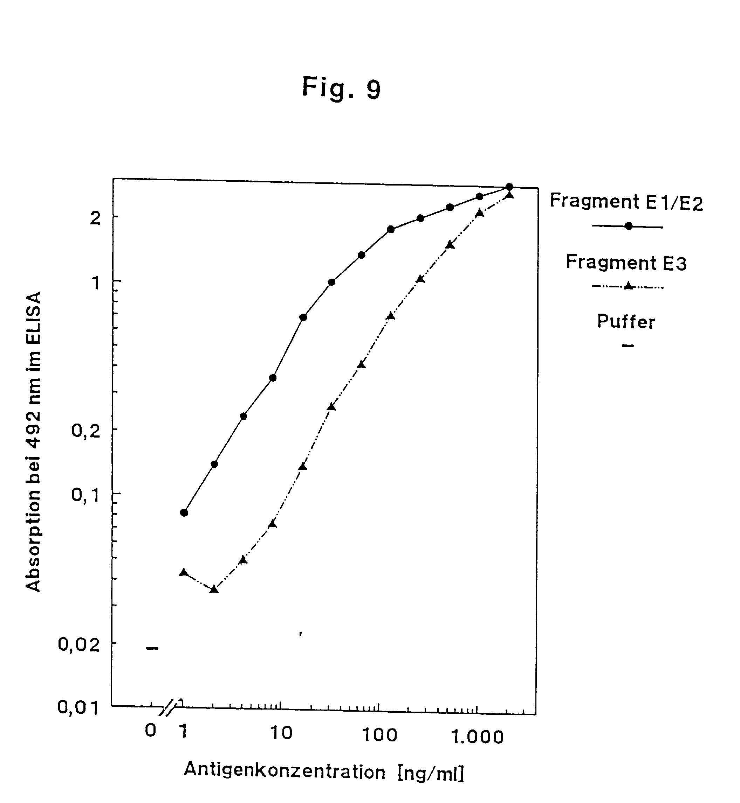

- Fig. 9:: Antikörper, gereinigt gegen RP4.

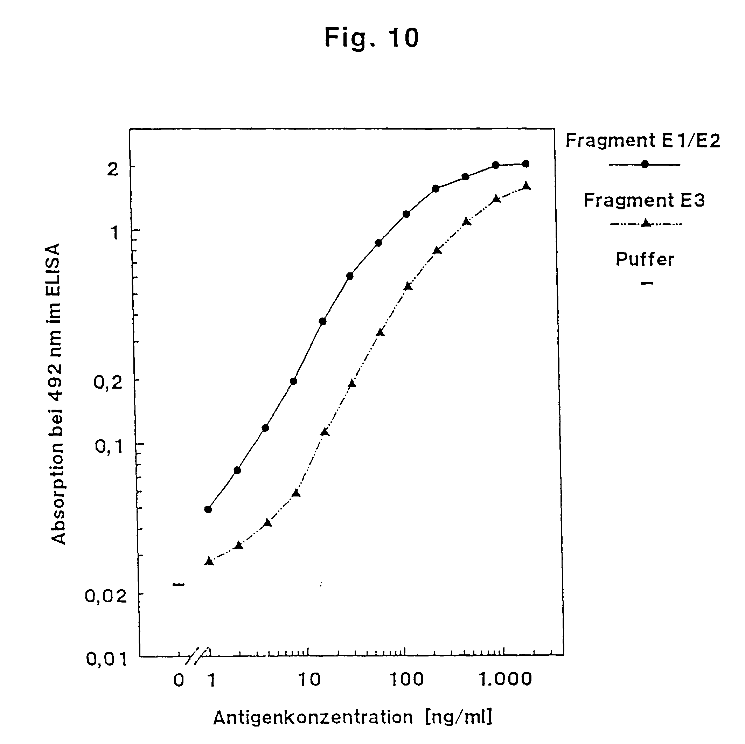

- Fig. 10:: Antikörper, gereinigt gegen RP5.

### Figuren 11 - 13:

Reaktion von Antikorpern aus Kaninchen gegen Peptid 3 mit Fragment E aus Fibrinogen (E3) in Probenpuffer und humanem Plasma im ELISA.

Fragment E, das durch Einwirkung von Plasmin auf Fibrinogen hergestellt wurde (= Fragment E3), wurde in verschiedenen Konzentrationen in Puffermedium bzw. in humanen Plasma verdünnt und die Reaktion mit den in der Legende zu den Figuren 1-5 beschriebenen Antikörpern im ELISA verfolgt. Dargestellt sind die Meßsignale im ELISA mit dem Antigen, sowie das Hintergrundsignal bei Verwendung von nicht mit Antigen aufgestocktem Puffer bzw. Plasma.
- Fig. 11:: Antikörper, gereinigt gegen RP1.

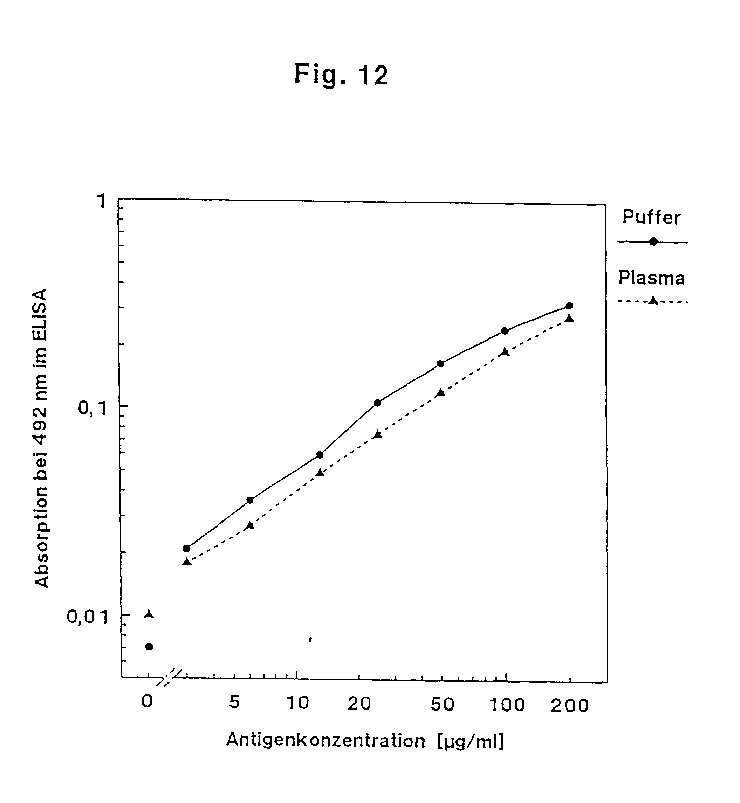

- Fig. 12:: Antikörper, gereinigt gegen RP2.

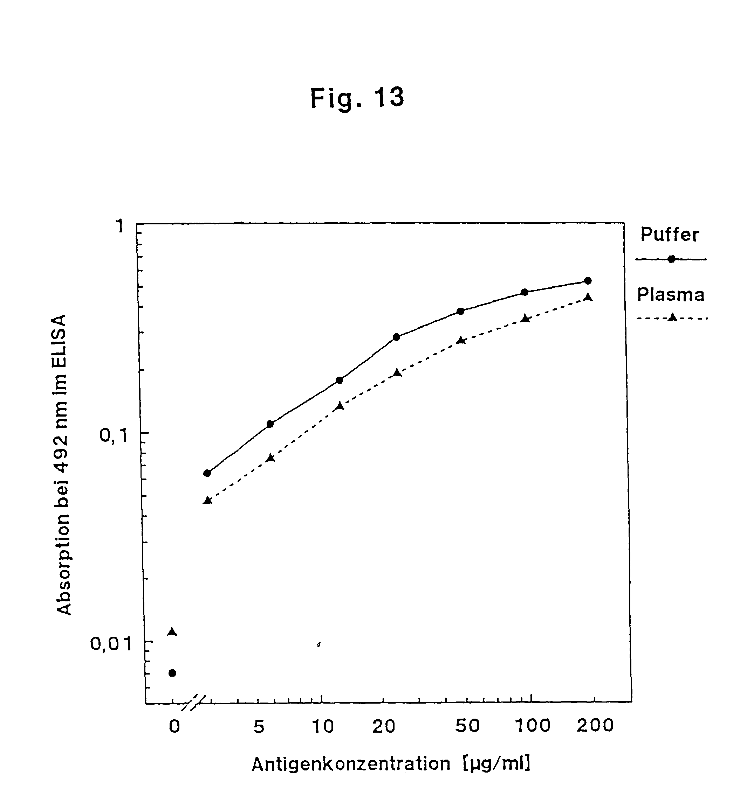

- Fig. 13:: Antikörper, gereinigt gegen RP3.

### Figuren 14 - 16:

Kinetik der in vitro Bildung von Fibrinogenspaltprodukten.

Humanes Plasma von normalen Blutspendern (SHP) und α2-Antiplasmin Mangelplasma (a2AP-MP) wurden mit gleichen Mengen an Plasmin versetzt und die Entstehung von Fibrinogenspaltprodukten im ELISA verfolgt. Auf der Festphase des ELISAs wurden die in der Legende zu den Abbildungen 1-5 beschriebenen Antikörper verwendet. Dargestellt sind die Meßsignale im ELISA nach verschieden langer Inkubation der Plasmen mit Plasmin.
- Fig. 14:: Antikörper, gereinigt gegen RP1.

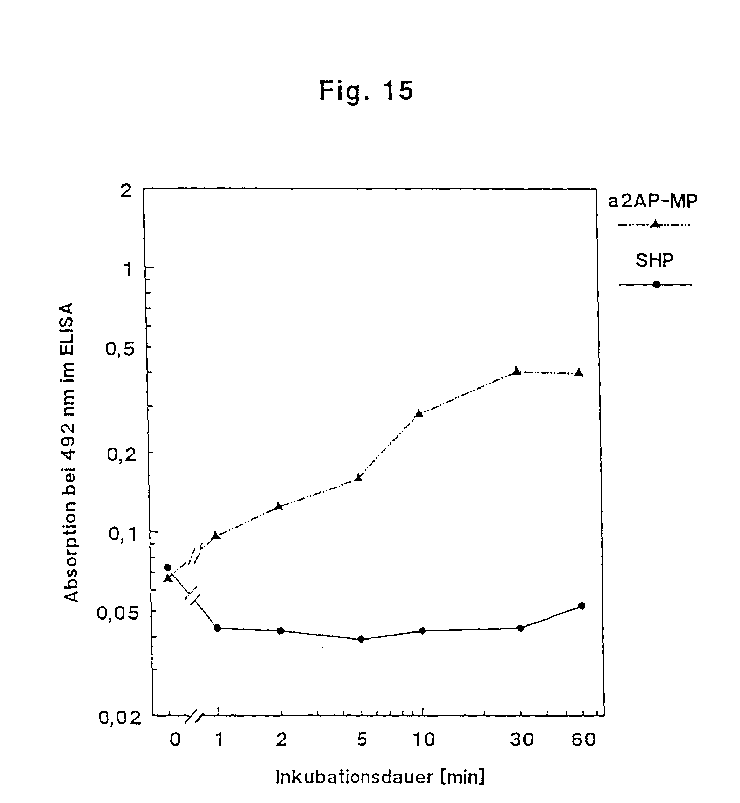

- Fig. 15:: Antikörper, gereinigt gegen RP2.

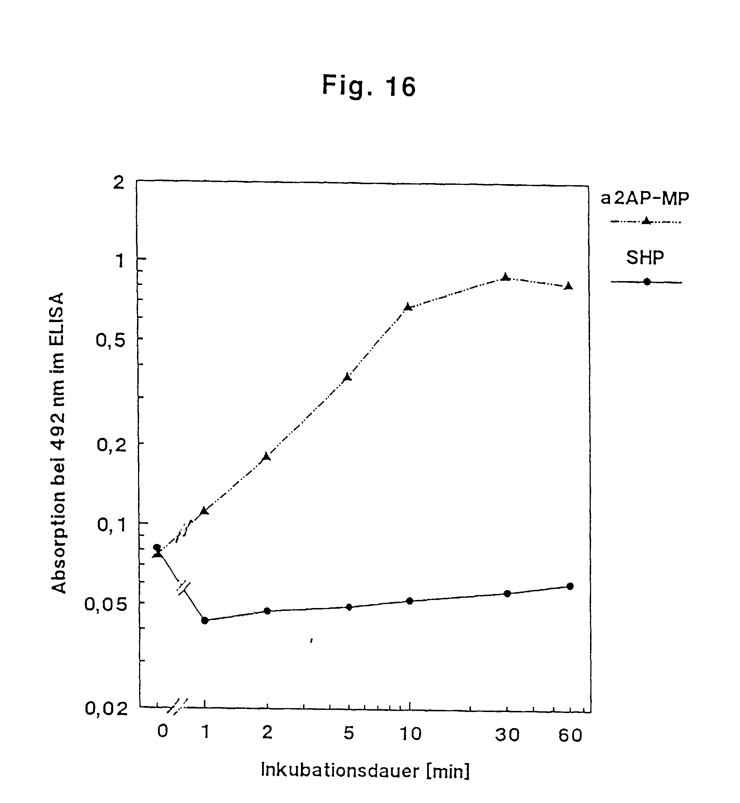

- Fig. 16:: Antikörper, gereinigt gegen RP3.

## Patentansprüche

1. Synthetische Peptide dadurch gekennzeichnet, daß sie an ihrem carboxyterminalen Ende eine der folgenden Aminosäuresequenzen aufweisen:

a) Leu-Phe-Glu-Tyr-Gln-Lys-OH;
b) Tyr-Met-Tyr-Leu-Leu-Lys-OH;
c) Val-Lys-Gln-Leu-Ile-Lys-OH und
d) His-Gln-Val-Glu-Asn-Lys-OH.

2. Synthetische Peptide nach Anspruch 1 dadurch gekennzeichnet, daß sie mindestens eine der folgenden Aminosäuresequenzen enthalten:

a) Asn-Lys-Leu-Lys-Asn-Ser-Leu-Phe-Gly-Tyr-Gln-Lys-OH,

b) Ser-Ser-Ser-Ser-Phe-Gln-Tyr-Met-Tyr-Leu-Leu-Lys-OH,

c) Glu-Asn-Lys-Thr-Ser-Gln-Val-Lys-Gln-Leu-Ile-Lys-OH
und

d) Ser-Leu-Glu-Asp-Ile-Leu-His-Gln-Val-Glu-Asn-Lys-OH.

3. Synthetische Peptide nach Anspruch 1, dadurch gekennzeichnet, daß sie mindestens eine der folgenden Aminosäuresequenzen enthalten:

a)His-Gln-Ser-Ala-Cys-Lys-Asp-Ser-Asp-Trp-Pro-Phe-Cys-
Ser-Asp-Glu-Asp-Trp-Asn-Tyr-Lys-Cys-Pro-Ser-Gly-Cys-
Arg-Met-Lys-Gly-Leu-Ile-Asp-Glu-Val-Asn-Gln-AspPhe-
Thr-Asn-Arg-Ile-Asn-Lys-Leu-Lys-Asn-Ser-Leu-Phe-Glu-
Tyr-Gln-Lys-OH (Peptid 1);

b) Lys-Val-Glu-Arg-Lys-Ala-Pro-Asp-Ala-Gly-Gly-Cys-Leu-
His-Ala-Asp-Pro-Asp-Leu-Gly-Val-Leu-Cys-Pro-Thr-Gly-
Cys-Gln-Leu-Gln-Glu-Ala-Leu-Leu-Gln-Gln-Glu-Arg-Pro-
Ile-Arg-Asn-Ser-Val-Asp-Glu-Leu-Asn-Asn-Asn-Val-Glu-
Ala-Val-Ser-Gln-Thr-Ser-Ser-Ser-Ser-Phe-Gln-Tyr-Met-
Tyr-Leu-Leu-Lys-OH (Peptid 2);

c)Tyr-Val-Ala-Thr-Arg-Asp-Asn-Cys-Cys-Ile-Leu-Asp-Glu-
Arg-Phe-Gly-Ser-Tyr-Cys-Pro-Thr-Thr-Cys-Gly-Ile-Ala-
Asp-Phe-Leu-Ser-Thr-Tyr-Gln-Thr-Lys-Val-Asp-Lys-Asp-
Leu-Gln-Ser-Leu-Glu-Asp-Ile-Leu-His-Gln-Val-Glu-Asn-
Lys-Thr-Ser-Glu-Val-Lys-Gln-Leu-Ile-Lys-OH
(Peptid 3) und

d)Tyr-Val-Ala-Thr-Arg-Asp-Asn-Cys-Cys-Ile-Leu-Asp-Glu-
Arg-Phe-Gly-Ser-Tyr-Cys-Pro-Thr-Thr-Cys-Gly-Ile-Ala-
Asp-Phe-Leu-Ser-Thr-Tyr-Gln-Thr-Lys-Val-Asp-Lys-Asp-
Leu-Gln-Ser-Leu-Glu-Asp-Ile-Leu-His-Gln-Val-Glu-Asn-
Lys (Peptid 4).

4. Peptide nach mindestens einem der Ansprüche 1 - 3 dadurch gekennzeichnet, daß sie direkt oder über einen Spacer an ein Trägermolekül gebunden sind.

5. Peptide nach mindestens einem der Ansprüche 1 - 3 dadurch gekennzeichnet, daß sie gentechnisch oder durch chemische Synthese hergestellt werden.

6. Antikörper dadurch gekennzeichnet, daß sie mit Peptiden gemäß Anspruch 1 immunchemisch reagieren.

7. Antikörper nach Anspruch 6 dadurch gekennzeichnet, daß sie durch Immunisierung eines Tieres mit einem Peptid gemäß mindestens einem der Ansprüche 1 - 5 erhalten werden.

8. Antikörper nach Anspruch 7 dadurch gekennzeichnet, daß sie über Immunadsorption an Peptide gemäß mindestens einem der Ansprüche 1 - 5 isoliert und gereinigt werden.

9. Antikörper nach Anspruch 7 dadurch gekennzeichnet, daß sie monoklonale Antikörper sind.

10. Diagnostisches Verfahren zur immunchemischen Bestimmung von intramolekularen Oligomeren aus Fibrinogen- und Fibrinspaltprodukten dadurch gekennzeichnet, daß Peptide nach Anspruch 1 und/oder Antikörper nach Anspruch 6 verwendet werden.

11. Verfahren nach Anspruch 10 dadurch gekennzeichnet, daß zur Bestimmung ein heterogener Immunoassay verwendet wird, bevorzugterweise ein Enzymimmunoassay.

12. Verfahren nach Anspruch 10 dadurch gekennzeichnet, daß zur Bestimmung ein homogener Immunoassay, bevorzugterweise ein partikelverstärkter, nephelometrischer oder turbidimetrischer Test verwendet wird.

13. Verfahren nach Anspruch 11 dadurch gekennzeichnet, daß ein Teil der Antikörper an eine Festphase gebunden ist und der andere Teil eine nachweisbare Funktion trägt.

14. Verfahren nach Anspruch 13 dadurch gekennzeichnet, daß als Festphase Mikrotitrationsplatten verwendet werden und die nachweisbare Funktion ein fluorogener oder lumineszenter Farbstoff oder ein Enzym ist.

15. Verfahren nach Anspruch 12 dadurch gekennzeichnet, daß als Festphase ein partikulärer wasserunlöslicher Träger verwendet wird, und die Agglutinationsreaktion mit Fibrin- und/oder Fibrinogenspaltprodukten nephelometrisch oder turbidimetrisch gemessen wird.

16. Verfahren nach Anspruch 10 dadurch gekennzeichnet, daß in dem Verfahren nur eine monospezifische Spezies verwendet wird.

17. Verfahren nach Anspruch 10 dadurch gekennzeichnet, daß der oder die Fangerantikorper Antikörper nach Anspruch 6 sind, während der oder die Detektionsantikörper davon verschieden sein können.

18. Verwendung von Peptiden nach mindestens einem der Ansprüche 1 - 5 zur Herstellung einer pharmazeutischen Zusammensetzung bestimmt zu therapeutischen Zwecken, insbesondere zur Therapie von Störungen des fibrinolytischen Systems.

19. Verwendung von Antikörpern nach mindestens einem der Ansprüche 6 - 9 zur Herstellung einer pharmazeutischen Zusammensetzung bestimmt zu therapeutischen Zwecken, inbesondere zur Therapie von Störungen des fibrinolytischen Systems.

20. Immunchemisches Verfahren zur Bestimmung von intramolekularen Oligomeren gemäß Anspruch 10 dadurch gekennzeichnet, daß nur eine monospezifische Antikorperspezies verwendet wird.

21. Verfahren nach Anspruch 20 dadurch gekennzeichnet, daß es ein heterogener Immunoassay ist, bevorzugterweise ein Enzymimmunoassay.

22. Verfahren nach Anspruch 20 dadurch gekennzeichnet, daß es ein homogener Immunoassay, bevorzugterweise ein partikelverstärkter, nephelometrischer oder turbidimetrischer Test ist.

23. Verfahren nach Anspruch 21 dadurch gekennzeichnet, daß ein Teil der Antikörper an eine Festphase gebunden ist und der andere Teil eine nachweisbare Funktion trägt.

24. Verfahren nach Anspruch 23 dadurch gekennzeichnet, daß als Festphase Mikrotitrationsplatten verwendet werden und die nachweisbare Funktion ein fluorogener oder lumineszenter Farbstoff oder ein Enzym ist.

## Claims

1. A synthetic peptide which has at its carboxyterminal end one of the following amino acid sequences:

a) Leu-Phe-Glu-Tyr-Gln-Lys-OH;

b) Tyr-Met-Tyr-Leu-Leu-Lys-OH;

c) Val-Lys-Gln-Leu-Ile-Lys-OH and

d) His-Gln-Val-Glu-Asn-Lys-OH.

2. A synthetic peptide as claimed in claim 1 which contains at least one of the following amino acid sequences:

a) Asn-Lys-Leu-Lys-Asn-Ser-Leu-Phe-Gly-Tyr-Gln-Lys-
OH,

b) Ser-Ser-Ser-Ser-Phe-Gln-Tyr-Met-Tyr-Leu-Leu-Lys-
OH,

c) Glu-Asn-Lys-Thr-Ser-Gln-Val-Lys-Gln-Leu-Ile-Lys-
OH and

d) Ser-Leu-Glu-Asp-Ile-Leu-His-Gln-Val-Glu-Asn-Lys-
OH.

3. A synthetic peptide as claimed in claim 1 which contains at least one of the following amino acid sequences:

a) His-Gln-Ser-Ala-Cys-Lys-Asp-Ser-Asp-Trp-Pro-Phe-
Cys-Ser-Asp-Glu-Asp-Trp-Asn-Tyr-Lys-Cys-Pro-Ser-
Gly-Cys-Arg-Met-Lys-Gly-Leu-Ile-Asp-Glu-Val-Asn-
Gln-Asp-Phe-Thr-Asn-Arg-Ile-Asn-Lys-Leu-Lys-Asn-
Ser-Leu-Phe-Glu-Tyr-Gln-Lys-OH (peptide 1);

b) Lys-Val-Glu-Arg-Lys-Ala-Pro-Asp-Ala-Gly-Gly-Cys-
Leu-His-Ala-Asp-Pro-Asp-Leu-Gly-Val-Leu-Cys-Pro-
Thr-Gly-Cys-Gln-Leu-Gln-Glu-Ala-Leu-Leu-Gln-Gln-
Glu-Arg-Pro-Ile-Arg-Asn-Ser-Val-Asp-Glu-Leu-Asn-
Asn-Asn-Val-Glu-Ala-Val-Ser-Gln-Thr-Ser-Ser-Ser-
Ser-Phe-Gln-Tyr-Met-Tyr-Leu-Leu-Lys-OH
(peptide 2);

c) Tyr-Val-Ala-Thr-Arg-Asp-Asn-Cys-Cys-Ile-Leu-Asp-
Glu-Arg-Phe-Gly-Ser-Tyr-Cys-Pro-Thr-Thr-Cys-Gly-
Ile-Ala-Asp-Phe-Leu-Ser-Thr-Tyr-Gln-Thr-Lys-Val-
Asp-Lys-Asp-Leu-Gln-Ser-Leu-Glu-Asp-Ile-Leu-His-
Gln-Val-Glu-Asn-Lys-Thr-Ser-Glu-Val-Lys-Gln-Leu-
Ile-Lys-OH (peptide 3) and

d) Tyr-Val-Ala-Thr-Arg-Asp-Asn-Cys-Cys-Ile-Leu-Asp-
Glu-Arg-Phe-Gly-Ser-Tyr-Cys-Pro-Thr-Thr-Cys-Gly-
Ile-Ala-Asp-Phe-Leu-Ser-Thr-Tyr-Gln-Thr-Lys-Val-
Asp-Lys-Asp-Leu-Gln-Ser-Leu-Glu-Asp-Ile-Leu-His-
Gln-Val-Glu-Asn-Lys (peptide 4).

4. A peptide as claimed in at least one of claims 1 - 3 which is bound to a carrier molecule either directly or via a spacer.

5. A peptide as claimed in at least one of claims 1 - 3 which is prepared by genetic manipulation or by chemical synthesis.

6. An antibody which reacts immunochemically with a peptide as claimed in claim 1.

7. An antibody as claimed in claim 6 which is obtained by immunizing an animal with a peptide as claimed in at least one of claims 1 - 5.

8. An antibody as claimed in claim 7 which is isolated and purified by means of immunoadsorption to a peptide as claimed in at least one of claims 1- 5.

9. An antibody as claimed in claim 7 which is a monoclonal antibody.

10. A diagnostic method for the immunochemical determination of intramolecular oligomers of cleavage products of fibrinogen and fibrin wherein peptides as claimed in claim 1 and/or antibodies as claimed in claim 6 are used.

11. The method as claimed in claim 10 wherein a heterogeneous immunoassay, preferably an enzyme immunoassay, is used for the determination.

12. The method as claimed in claim 10 wherein a homogeneous immunoassay, preferably a particle-boosted, nephelometric or turbidimetric test, is used for the determination.

13. The method as claimed in claim 11 wherein one part of the antibodies is bound to a solid phase and the other part carries a detectable function.

14. The method as claimed in claim 13 wherein microtitration plates are used as the solid phase and the detectable function is a fluorogenic or luminescent dye or an enzyme.

15. The method as claimed in claim 12 wherein a particulate, water-insoluble support is used as the solid phase and the agglutination reaction with cleavage products of fibrin and/or fibrinogen is measured nephelometrically or turbidimetrically.

16. The method as claimed in claim 10 wherein only one monospecific species is used in the method.

17. The method as claimed in claim 10 wherein the capture antibody(ies) is/are (an) antibody(ies) as claimed in claim 6 while the detection antibody(ies) can be different therefrom.

18. The use of peptides as claimed in at least one of claims 1 - 5 for the preparation of a pharmaceutical composition intended for therapeutic purposes, in particular for the therapy of disturbances of the fibrinolytic system.

19. The use of antibodies as claimed in at least one of claims 6 - 9 for the preparation of a pharmaceutical composition intended for therapeutic purposes, in particular for the therapy of disturbances of the fibrinolytic system.

20. An immunochemical method for determining intramolecular oligomers as claimed in claim 10 wherein only one monospecific antibody species is used.

21. The method as claimed in claim 20 which is a heterogeneous immunoassay, preferably an enzyme immunoassay.

22. The method as claimed in claim 20 which is a homogeneous immunoassay, preferably a particle-boosted, nephelometric or turbidimetric test.

23. The method as claimed in claim 21 wherein one part of the antibodies is bound to a solid phase and the other part carries a detectable function.

24. The method as claimed in claim 23 wherein microtitration plates are used as the solid phase and the detectable function is a fluorogenic or luminescent dye or an enzyme.

## Revendications

1. Peptides synthétiques, caractérisés en ce qu'ils présentent à leur extrémité carboxy-terminale l'une des séquences d'aminoacides suivantes

a) Leu-Phe-Glu-Tyr-Gln-Lys-OH,
b) Tyr-Met-Tyr-Leu-Leu-Lys-OH,
c) Val-Lys-Gln-Leu-Ile-Lys-OH et
d) His-Gln-Val-Glu-Asn-Lys-OH.

2. Peptides synthétiques selon la revendication 1, caractérisés en ce qu'ils comportent au moins l'une des séquences d'aminoacides suivantes:

a) Asn-Lys-Leu-Lys-Asn-Ser-Leu-Phe-Gly-Tyr-Gln-Lys-OH,

b) Ser-Ser-Ser-Ser-Phe-Gln-Tyr-Met-Tyr-Leu-Leu-Lys-OH,

c) Glu-Asn-Lys-Thr-Ser-Gln-Val-Lys-Gln-Leu-Ile-Lys-OH
   et

d) Ser-Leu-Glu-Asp-Ile-Leu-His-Gln-Val-Glu-Asn-Lys-OH.

3. Peptides synthétiques selon la revendication 1, caractérisés en ce qu'ils comportent au moins l'une des séquences d'aminoacides suivantes:

a) His-Gln-Ser-Ala-Cys-Lys-Asp-Ser-Asp-Trp-Pro-Phe-Cys-
Ser-Asp-Glu-Asp-Trp-Asn-Tyr-Lys-Cys-Pro-Ser-Gly-Cys-
Arg-Met-Lys-Gly-Leu-Ile-Asp-Glu-Val-Asn-Gln-Asp-Phe-
Thr-Asn-Arg-Ile-Asn-Lys-Leu-Lys-Asn-Ser-Leu-Phe-Glu-
Tyr-Gln-Lys-OH (peptide 1),

b) Lys-Val-Glu-Arg-Lys-Ala-Pro-Asp-Ala-Gly-Gly-Cys-Leu-
His-Ala-Asp-Pro-Asp-Leu-Gly-Val-Leu-Cys-Pro-Thr-Gly-
Cys-Gln-Leu-Gln-Glu-Ala-Leu-Leu-Gln-Gln-Glu-Arg-Pro-
Ile-Arg-Asn-Ser-Val-Asp-Glu-Leu-Asn-Asn-Asn-Val-Glu-
Ala-Val-Ser-Gln-Thr-Ser-Ser-Ser-Ser-Phe-Gln-Tyr-Met-
Tyr-Leu-Leu-Lys-OH (peptide 2),

c) Tyr-Val-Ala-Thr-Arg-Asp-Asn-Cys-Cys-Ile-Leu-Asp-Glu-
Arg-Phe-Gly-Ser-Tyr-Cys-Pro-Thr-Thr-Cys-Gly-Ile-Ala-
Asp-Phe-Leu-Ser-Thr-Tyr-Gln-Thr-Lys-Val-Asp-Lys-Asp-
Leu-Gln-Ser-Leu-Glu-Asp-Ile-Leu-His-Gln-Val-Glu-Asn-
Lys-Thr-Ser-Glu-Val-Lys-Gln-Leu-Ile-Lys-OH (peptide 3)
et

d) Tyr-Val-Ala-Thr-Arg-Asp-Asn-Cys-Cys-Ile-Leu-Asp-Glu-
Arg-Phe-Gly-Ser-Tyr-Cys-Pro-Thr-Thr-Cys-Gly-Ile-Ala-
Asp-Phe-Leu-Ser-Thr-Tyr-Gln-Thr-Lys-Val-Asp-Lys-Asp-
Leu-Gln-Ser-Leu-Glu-Asp-Ile-Leu-His-Gln-Val-Glu-Asn-
Lys (peptide 4).

4. Peptides selon au moins l'une des revendications 1 à 3, caractérisés en ce qu'ils sont liés à une molécule porteuse, directement ou par l'intermédiaire d'un espaceur.

5. Peptides selon au moins l'une des revendications 1 à 3, caractérisés en ce qu'ils sont produits par génie génétique ou par synthèse chimique.

6. Anticorps, caractérisés en ce qu'ils réagissent immunochimiquement avec des peptides selon la revendication 1.

7. Anticorps selon la revendication 6, caractérisés en ce qu'ils sont obtenus par immunisation d'un animal avec un peptide selon au moins l'une des revendications 1 à 5.

8. Anticorps selon la revendication 7, caractérisés en ce qu'ils sont isolés et purifiés par immuno-adsorption sur des peptides selon au moins l'une des revendications 1 à 5.

9. Anticorps selon la revendication 7, caractérisés en ce qu'ils sont des anticorps monoclonaux.

10. Procédé de diagnostic pour la détermination immunochimique d'oligomères intramoléculaires constitués de produits de coupure de la fibrine et du fibrinogène, caractérisé en ce que l'on utilise des peptides selon la revendication 1 et/ou des anticorps selon la revendication 6.

11. Procédé selon la revendication 10, caractérisé en ce que l'on utilise pour la détermination un essai immunologique hétérogène, de préférence un essai immuno-enzymatique.

12. Procédé selon la revendication 10, caractérisé en ce que l'on utilise pour la détermination un essai immunologique homogène, de préférence un essai néphélométrique ou turbidimétrique renforcé par des particules.

13. Procédé selon la revendication 11, caractérisé en ce qu'une partie des anticorps est fixée à une phase solide et l'autre partie porte une fonction détectable.

14. Procédé selon la revendication 13, caractérisé en ce que l'on utilise comme phase solide des plaques de microtitrage et la fonction détectable est un colorant fluorescent ou luminescent ou une enzyme.

15. Procédé selon la revendication 12, caractérisé en ce que l'on utilise comme phase solide un support particulaire insoluble dans l'eau, et la réaction d'agglutination avec des produits de coupure de la fibrine et/ou du fibrinogène est mesurée par néphélométrie ou turbidimétrie.

16. Procédé selon la revendication 10, caractérisé en ce que l'on n'utilise dans le procédé qu'une seule espèce monospécifique.

17. Procédé selon la revendication 10, caractérisé en ce que le ou les anticorps capteurs sont des anticorps selon la revendication 6, tandis que le ou les anticorps de détection peuvent être différents de ceux-ci.

18. Utilisation de peptides selon au moins l'une des revendications 1 à 5, pour la préparation d'une composition pharmaceutique destinée à des fins thérapeutiques, en particulier au traitement de troubles du système fibrinolytique.

19. Utilisation d'anticorps selon au moins l'une des revendications 6 à 9, pour la préparation d'une composition pharmaceutique destinée à des fins thérapeutiques, en particulier au traitement de troubles du système fibrinolytique.

20. Procédé immunochimique pour la détermination d'oligoméres intramoléculaires selon la revendication 10, caractérisé en ce que l'on n'utilise qu'une seule espèce d'anticorps monospécifique.

21. Procédé selon la revendication 20, caractérisé en ce qu'il s'agit d'un essai immunologique hétérogène, de préférence d'un essai immunoenzymatique.

22. Procédé selon la revendication 20, caractérisé en ce qu'il s'agit d'un essai immunologique hétérogène, de préférence d'un essai néphélométrique ou turbidimétrique renforcé par des particules.

23. Procédé selon la revendication 21, caractérisé en ce qu'une partie des anticorps est fixée à une phase solide et l'autre partie porte une fonction détectable.

24. Procédé selon la revendication 23, caractérisé en ce que l'on utilise comme phase solide des plaques de microtitrage, et la fonction détectable est un colorant fluorescent ou luminescent ou une enzyme.
